# EUROPÄISCHE PATENTSCHRIFT

(11) **EP 2 502 599 B1**
(45) Veröffentlichungstag und Bekanntmachung des Hinweises auf die Patenterteilung: **08.06.2016**
(21) Anmeldenummer: 12159846.0
(22) Anmeldetag: 16.03.2012
(51) Int. Cl.: A61C 5/06, B05C 17/01, B65D 83/00, B05C 17/005, A61B 17/88, A61M 5/315

(54) **Drehspritze sowie Spritzenkörper und Mutterteil für eine Drehspritze**
Rotating syringe and syringe body and screw nut for a rotating syringe
Seringue à vis ainsi que corps de seringue et écrou pour seringue à vis

(30) Priorität: 22.03.2011 DE 102011005919
(43) Veröffentlichungstag der Anmeldung: 26.09.2012
(73) Patentinhaber: VOCO GmbH, 27472 Cuxhaven (DE)
(72) Erfinder: Leiner, Uwe, 27632 Midlum (DE); Plaumann, Manfred Thomas, 27476 Cuxhaven (DE)
(74) Vertreter: Eisenführ Speiser

(56) Entgegenhaltungen:
- WO-A1-2005/097239
- DE-C1- 19 833 238
- US-A- 5 618 273
- US-A1- 2002 010 431
- US-A1- 2006 131 344

## Beschreibung

Die Erfindung betrifft eine Drehspritze zur Abgabe eines Werkstoffs, insbesondere einer pastösen und/oder fließfähigen Dentalsubstanz, umfassend eine Kolbenspindel mit einem Außengewinde, einen Spritzenkörper mit einer Längsachse, umfassend eine Hülse zur Aufnahme eines Werkstoffs, wobei die Hülse ein Austrittsende für den Austritt eines in der Hülse befindlichen Werkstoffs in einer axialen Austrittsrichtung und ein Kolbenende zur Aufnahme der Kolbenspindel aufweist, und ein Mutterteil mit einem Innengewinde für einen Eingriff mit dem Außengewinde der Kolbenspindel, wobei das Mutterteil in einer radialen Aufschieberichtung auf das Kolbenende des Spritzenkörpers aufgeschoben ist.

Die Erfindung betrifft ferner einen Spritzenkörper für eine Drehspritze zur Abgabe eines Werkstoffs der eingangs genannten Art, umfassend eine Hülse zur Aufnahme eines Werkstoffs, wobei die Hülse ein Austrittsende für den Austritt eines in der Hülse befindlichen Werkstoffs in einer axialen Austrittsrichtung und ein Kolbenende zur Aufnahme einer Kolbenspindel aufweist, und wobei das Kolbenende derart ausgebildet ist, dass ein Mutterteil für eine Drehspritze der eingangs genannten Art in eine radiale Aufschieberichtung auf das Kolbenende aufschiebbar ist.

Die Erfindung betrifft ferner ein Mutterteil für eine Drehspritze zur Abgabe eines Werkstoffs der eingangs genannten Art, umfassend ein Innengewinde für einen Eingriff mit einem Außengewinde einer Kolbenspindel, wobei das Mutterteil derart ausgebildet ist, dass es in eine radiale Aufschieberichtung auf ein Kolbenende eines Spritzenkörpers für eine Drehspritze der eingangs genannten Art aufschiebbar ist.

Drehspritzen der eingangs genannten Art werden zur Abgabe, insbesondere einer dosierten, Abgabe eines Werkstoffs eingesetzt. Als Werkstoffe kommen insbesondere (aber nicht ausschließlich) fließfähige und/oder pastöse Dentalsubstanzen oder dentale Werkstoffe in Betracht. Im Dentalbereich werden verschiedene Materialien in unterschiedlichen Anwendungen eingesetzt. Ein Beispiel sind als Komposite bezeichnete zahnfarbene plastische Füllungsmaterialien mit einer Kunststoffmatrix und Füllkörpern. Komposite können eine hochviskose, stopfbare Konsistenz bis hin zu einer niedrigviskosen, fließfähigen Konsistenz aufweisen.

Dentale Werkstoffe wie Komposite werden üblicherweise in vorgefüllten Drehspritzen in Verkehr gebracht. Eine solche vorgefüllte Spritze enthält in einer vorzugsweise ganz oder teilweise hohlzylinderförmig ausgebildeten Hülse des Spritzenkörpers den abzugebenden Werkstoff. Durch Betätigung der Kolbenspindel wird der Werkstoff in einer axialen Austrittsrichtung aus dem Austrittsende der Hülse ausgetrieben, indem beim Drehen der Kolbenspindel durch den Eingriff des Außengewindes der Kolbenspindel mit dem Innengewinde des Mutterteils die Kolbenspindel in die mit Werkstoff gefüllte Hülse des Spritzenkörpers vordringt und so den Werkstoff aus der Hülse austreibt.

Auch in anderen Anwendungsbereichen können fließfähige und/oder pastöse Werkstoffe aus einer Drehspritze abgegeben werden, beispielsweise beim Löten, beim Aufbringen von Pasten oder beim Applizieren von Klebstoffen oder Dichtungsmaterialien.

Um die Kolbenspindel in der Hülse des Spritzenkörpers entlang der axialen Austrittsrichtung vortreiben zu können, muss das Innengewinde des Mutterteils einen kleineren Durchmesser haben als die Hülse des Spritzenkörpers. Das Mutterteil mit dem Innengewinde ist daher am Spritzenkörper anzubringen beziehungsweise zu montieren. Eine Schraubverbindung zwischen Mutterteil und Spritzenkörper hat den Nachteil, dass beim Betätigen der Kolbenspindel ungewollt die Schraubverbindung zwischen Spritzenkörper und Mutterteil gelöst werden kann.

Eine andere Verbindungsmöglichkeit ist ein Steckmechanismus, bei dem das Mutterteil in einer radialen Aufschieberichtung auf das Kolbenende des Spritzenkörpers aufgeschoben wird. Als Kolbenende des Spritzenkörpers wird hier ein Abschnitt an dem Ende des Spritzenkörpers bezeichnet, in den die Kolbenspindel eindringt, das heißt, ein Bereich an dem Ende des Spritzenkörpers, der dem Austrittsende der Hülse gegenüberliegt. Auch Drehspritzen mit Steckmechanismus sind jedoch weiter verbesserungsfähig, insbesondere hinsichtlich der Zuverlässigkeit der Verbindung zwischen Mutterteil und Spritzenkörper und hinsichtlich der Vereinfachung des Montagevorganges.

Dokument WO 2005/097239 beispielweise offenbart eine Drehspritze zur Abgabe einer Medizinsubstanz, umfassend eine Kolbenspindel mit einem Außengewinde, einen Spritzenkörper mit einer Hülse zur Aufnahme eines Werkstoffs, und einen Griff mit einem Innengewinde für einen Eingriff mit dem Außengewinde der Kolbenspindel. Der Spritzenkörper weist eine Schulter an seinem Rückende auf und der Griff ist in einer radialen Aufschieberichtung auf diese Schulter aufgeschoben, sodass eine Axialverschiebung des Griffs gegenüber dem Spritzenkörper verhindert ist.

Es ist eine Aufgabe der Erfindung, eine Drehspritze und einen Spritzenkörper sowie ein Mutterteil für eine Drehspritze anzugeben, die einen gegenüber bekannten Drehspritzen verbesserten Steckmechanismus bzw. Komponenten für einen solchen Steckmechanismus aufweisen. Eine weitere Aufgabe der Erfindung ist es, eine Drehspritze und einen Spritzenkörper sowie ein Mutterteil für eine Drehspritze anzugeben, welche ein ungewolltes Lösen der Verbindung von Mutterteil und Spritzenkörper und/oder eine ungewollte Verformung eines oder mehrerer Teile der Drehspritze, insbesondere des Mutterteils, verhindern oder erschweren. Es ist ferner eine Aufgabe der Erfindung, eine Drehspritze und einen Spritzenkörper sowie ein Mutterteil für eine Drehspritze anzugeben, die unter hygienischen Gesichtspunkten verbessert sind. Es ist eine weitere Aufgabe der Erfindung, eine Drehspritze und einen Spritzenkörper sowie ein Mutterteil für eine Drehspritze anzugeben, deren Handhabung vereinfacht oder verbessert ist. Eine weitere Aufgabe ist es, den Montagevorgang zu vereinfachen. Ferner ist es eine Aufgabe der Erfindung, eine Drehspritze und einen Spritzenkörper sowie ein Mutterteil für eine Drehspritze anzugeben, die eine zuverlässigere Befestigung des Mutterteils am Spritzenkörper gewährleisten.

Die Aufgabe wird gelöst durch ein Drehspritze nach Anspruch 1.

Bei einer erfindungsgemäßen Drehspritze ist das Mutterteil nach dem Aufschieben auf das Kolbenende des Spritzenkörpers in axialer Richtung nicht gegenüber dem Spritzenkörper verschieblich: eine Axialverschiebung des Mutterteils gegenüber dem Spritzenkörper in Austrittsrichtung ist durch einen Formschluss des Flansches des Mutterteils mit einer Gegenfläche am Kolbenende verhindert und eine Axialverschiebung des Mutterteils gegenüber dem Spritzenkörper entgegen der Austrittsrichtung ist durch einen Formschluss von am Mutterteil ausgebildeten Halteflächen mit am Kolbenende ausgebildeten Gegenhalteflächen verhindert. Die Gegenfläche stellt somit einen Anschlag für den Flansch dar, so dass das Mutterteil in Austrittsrichtung gegenüber dem Spritzenkörper nicht verschieblich ist. Ebenso stellen die Halteflächen mit den Gegenhalteflächen einen weiteren Anschlag dar, der eine Verschiebung des Mutterteils entgegen der Austrittsrichtung verhindert.

Besonders bevorzugt ist ein Formschluss eines Paares von am Mutterteil ausgebildeten Halteflächen mit einem Paar von am Kolbenende ausgebildeten Gegenhalteflächen. Die Halteflächen des Mutterteils können vorzugsweise an einem Paar von in axiale Richtung weisenden, vom Flansch des Mutterteils abgehenden Armen ausgebildet sein.

Bei einer erfindungsgemäßen Drehspritze ist nach dem Aufschieben des Mutterteils auf das Kolbenende des Spritzenkörpers zudem eine Relativbewegung zwischen den Halteflächen und den Gegenhalteflächen in einer radialen Richtung, die senkrecht zur Aufschieberichtung gerichtet ist, durch einen Formschluss zwischen den Halteflächen und den Gegenhalteflächen verhindert.

Der Formschluss ist erfindungsgemäß durch zur Längsachse geneigte ausgebildete Halteflächen und Gegenhalteflächen. Der Formschluss kann auch derart realisiert sein, dass die Halteflächen und Gegenhalteflächen abschnittsweise unterschiedliche Neigungen, Krümmungen, Hinterschnitte und/oder Stufen aufweisen.

Ein Vorteil dieser Ausgestaltung der Halteflächen und Gegenhalteflächen ist es, dass durch den Formschluss auch ein radiales Abrutschen des Mutterteils vom Spritzenkörper senkrecht zur Aufschieberichtung erschwert oder verhindert wird. Auf diese Weise wird eine zuverlässigere Befestigung des Mutterteils am Spritzenkörper erzielt, insbesondere auch bei Einwirkung von durch die Betätigung der Kolbenspindel zum Austreiben von in der Hülse befindlichem Werkstoff auf den Spritzenkörper und das Mutterteil aufgebrachten Kräften, insbesondere Axialkräften.

Diese Axialkräfte werden von der Spindel über den Flansch des Mutterteils - und in Ausführungsformen, in denen die Halteflächen an Armen ausgebildet sind, noch über diese Arme - in die Halteflächen geleitet. Insbesondere in Ausführungsformen, in denen die Arme seitlich versetzt zu den Halteflächen angeordnet sind, entsteht an dieser Stelle ein Moment, das bestrebt ist, die Arme nach außen aufzubiegen bzw. aufzuspreizen. Die Ausgestaltung der Halteflächen und Gegenhalteflächen mit einem Formschluss, der eine radiale, senkrecht zur Aufschieberichtung gerichtete Relativbewegung zwischen den Halteflächen und den Gegenhalteflächen verhindert, wirkt dem entgegen.

Im Lager- und Vertriebszustand weist die Drehspritze vorzugsweise eine abnehmbare Kappe auf, mit der das Austrittsende bis zum bestimmungsgemäßen Gebrauch verschlossen werden kann.

Die erfindungsgemäße Drehspritze wird vorzugsweise im Spritzgießverfahren hergestellt, wobei vorzugsweise Kolbenspindel, Spritzenkörper, Mutterteil und ggf. Kappe getrennt voneinander hergestellt und das Mutterteil auf das Kolbenende des Spritzenkörpers in radialer Aufschieberichtung aufgeschoben wird. Die Kolbenspindel wird in das Innengewinde des am Kolbenende des Spritzenkörpers befindlichen Mutterteils nur soweit eingedreht, dass im Lager- und Vertriebszustand ein in der Hülse des Spritzenkörpers befindlicher Werkstoff nicht aus dem Austrittsende ausgetrieben wird. Erst beim bestimmungsgemäßen Gebrauch der Drehspritze durch den Anwender wird die Kolbenspindel weiter in die Hülse eingedreht und dadurch der in der Hülse befindliche Werkstoff aus dem Austrittsende ausgetrieben.

Die Halteflächen und Gegenhalteflächen sind derart gegenüber der Längsachse geneigt, dass eine radiale, senkrecht zur Aufschieberichtung gerichtete Relativbewegung zwischen den Halteflächen und den Gegenhalteflächen verhindert ist.

Eine bevorzugte Ausführungsform ist dadurch gekennzeichnet, dass die Halteflächen und Gegenhalteflächen derart gegenüber der Längsachse hinterschnitten sind, dass eine radiale, senkrecht zur Aufschieberichtung gerichtete Relativbewegung zwischen den Halteflächen und den Gegenhalteflächen verhindert ist.

In dieser Ausführungsform ist nach dem Aufschieben des Mutterteils auf das Kolbenende des Spritzenkörpers eine Relativbewegung zwischen den Halteflächen und den Gegenhalteflächen in einer radialen Richtung, die senkrecht zur Aufschieberichtung gerichtet ist, dadurch verhindert, dass die Halteflächen und Gegenhalteflächen gegenüber der Längsachse geneigt und/oder hinterschnitten sind.

Die Halteflächen und Gegenhalteflächen schließen einen Winkel mit der Längsachse ein, der größer als 0 ° und kleiner als 90° ist. Vorzugsweise liegt der Winkel in einem Bereich, dessen untere Grenze 5, 10, 15, 20, 25, 30, 35, 40 oder 45 ° beträgt und dessen obere Grenze vorzugsweise 85, 80, 75 , 70, 65, 60, 55, 50 oder 45° beträgt. Insbesondere bevorzugt ist ein Winkel von 70°. Die genaue Auswahl des Winkels trifft der Fachmann anhand der vorliegenden Material- Reibungs- und Elastizitätsparameter der eingesetzten Spritzenwerkstoffe sowie anhand von mit der äußeren Spritzenform verbundenen geometrischen Platzverhältnissen.

Eine hinterschnittene Ausbildung der Halteflächen und Gegenhalteflächen gewährleistet in besonders zuverlässiger und damit vorteilhafter Weise eine Sicherung gegen eine radiale, senkrecht zur Aufschieberichtung gerichtete Relativbewegung zwischen den Halteflächen und den Gegenhalteflächen und verhindert damit zuverlässig ein Abrutschen der am Mutterteil ausgebildeten Halteflächen von den am Kolbenende des Spritzenkörpers ausgebildeten Gegenhalteflächen in einer radialen Richtung senkrecht zur Aufschieberichtung.

Eine weitere bevorzugte Ausführungsform ist dadurch gekennzeichnet, dass die Halteflächen und Gegenhalteflächen derart stufenförmig ausgebildet sind, dass eine radiale, senkrecht zur Aufschieberichtung gerichtete Relativbewegung zwischen den Halteflächen und den Gegenhalteflächen verhindert ist.

Ein Formschluss in senkrecht zur Aufschieberichtung liegender Richtung kann insbesondere auch durch einen oder mehrere stufenförmige, zum Beispiel rechtwinklige, Absätze erreicht werden.

Die Halteflächen und Gegenhalteflächen bilden derart einen Formschluss, dass ein durch eine bei Betätigung der Kolbenspindel aufgebrachte Kraft verursachtes Aufspreizen des Mutterteils oder Teilen davon nach außen verhindert ist.

Beim Betätigen der Kolbenspindel durch Eindrehen des Außengewindes der Kolbenspindel in das Innengewinde des Mutterteils treibt die Kolbenspindel in der Hülse befindlichen Werkstoff gegen eine zwischen der Hülseninnenwandung und dem Werkstoff bestehende Haft- bzw. Gleitreibung aus dem Austrittsende der Hülse aus. Dabei wird eine Axialkraft auf Mutterteil und Spritzenkörper ausgeübt, die bei nicht erfindungsgemäß ausgebildeten Drehspritzen zu einer Verformung des Mutterteils oder Teilen davon führen kann, die zu einem Aufbiegen des Mutterteils oder Teilen davon in eine im Wesentlichen radial nach außen gerichtete Richtung führen kann. Eine solche auch als Aufspreizen bezeichnete Verformung ist unerwünscht, da sie zu einer Beschädigung des Mutterteils und/oder des Spritzenkörpers und/oder zu einem Lösen der Verbindung zwischen Mutterteil und Spritzenkörper führen kann. Bei Halte- und Gegenhaltflächen, die keinen Formschluss bilden, ist die einzige Möglichkeit zur Verhinderung des Abrutschens die entsprechend breite Ausführung von Halte- und Gegenhalteflächen. Das hat den Nachteil, dass die Gestaltungsmöglichkeiten für das Kolbenende stark eingeschränkt sind. In Ausführungsformen, in denen die Halteflächen und Gegenhalteflächen an Armen ausgebildet sind, müssen dann die Arme entsprechend weit auseinander liegen, wodurch in nachteiliger Weise starke Verformungen und Materialbelastungen des Flansches durch die einwirkenden Kräfte zu erwarten sind.

Durch einen entsprechend ausgebildeten Formschluss der Halteflächen und Gegenhalteflächen kann ein solches Aufspreizen des Mutterteils oder Teilen davon nach außen einfach und wirksam verhindert werden. Dies ist insbesondere in Ausführungsformen von Vorteil, bei denen die Halteflächen des Mutterteils an einem Paar von in axialer Richtung weisenden, vom Flansch des Mutterteils abgehenden Armen ausgebildet sind.

In einer bevorzugten Ausführungsform bilden die Halteflächen und Gegenhalteflächen derart einen Formschluss, dass beim Aufbringen einer Axialkraft durch eine Betätigung der Kolbenspindel an den Halteflächen und Gegenhalteflächen Kraftkomponenten in einer Richtung wirken, die in einer zur Aufschieberichtung im Wesentlichen senkrechten Ebene liegen.

Durch einen entsprechend ausgebildeten Formschluss der Halteflächen und Gegenhalteflächen wirken bei einer durch Spindelbetätigung aufgebrachten Axialkraft an den Halteflächen und Gegenhalteflächen auch Kraftkomponenten in einer Richtung, die in einer Ebene liegt, zu der die Aufschieberichtung senkrecht steht. Insbesondere wirken die Kraftkomponenten auch in einer radialen Richtung, die im Wesentlichen senkrecht zur Aufschieberichtung und im Wesentlichen senkrecht zur Längsachse liegt. Diese Kraftkomponenten wirken einer radialen, senkrecht zur Aufschieberichtung gerichteten Relatiwerschiebung der Halte- und Gegenhalteflächen, insbesondere einer Aufspreizung des Mutterteils oder Teilen davon nach außen, entgegen.

In einer bevorzugten Ausführungsform weisen das Kolbenende und das Mutterteil jeweils mindestens eine Anschlagfläche auf, die derart ausgebildet und angeordnet sind, dass sie in Aufschieberichtung einen Anschlag für das Mutterteil bilden. Diese Art der Begrenzung des Aufschiebeweges bringt besonders bei der Montage von Hand, aber auch bei maschineller Montage des Mutterteils den Vorteil, dass ein zu weites Aufschieben und eine genaue Kontrolle dieses Umstandes entfallen kann.

In dieser Ausführungsform wird durch die Anschlagflächen eine Position des Mutterteils auf dem Kolbenende definiert, in der ein weiteres Aufschieben des Mutterteils in Aufschieberichtung nicht mehr möglich ist, d.h. es wird eine Endposition des Mutterteils auf den Spritzenkörper definiert. Dabei ist es bevorzugt, dass in dieser Endposition das Innengewinde des Mutterteils koaxial mit der Hülse des Spritzenkörpers ausgerichtet ist, so dass die in das Innengewinde eingedrehte Kolbenspindel ebenfalls koaxial in der Hülse verläuft.

Die Definition dieser Endposition des Mutterteils über die Anschlagflächen hat den Vorteil, dass beim Aufschieben des Mutterteils auf den Spritzenkörper die gewünschte Endposition des Mutterteils auf einfache Art und Weise sichergestellt werden kann und sowohl ein über die korrekte Position hinausgehendes Aufschieben durch den Anschlag wirksam verhindert ist. Ebenfalls kann ein Stoppen des Aufschiebevorgangs noch vor Erreichen der gewünschte Endposition des Mutterteils verhindert werden, da durch den Anschlag keine Gefahr besteht, dass das Mutterteil zu weit aufgeschoben wird und es daher immer bis zum Anschlag aufgeschoben werden kann.

In einer bevorzugten Ausführungsform weist das Kolbenende und/oder das Mutterteil einen Verbindungsmechanismus auf, der ein Abziehen des Mutterteils vom Kolbenende entgegen der Aufschieberichtung verhindert oder erschwert.

Ein solcher Verbindungsmechanismus verhindert oder erschwert, dass sich das Mutterteil aus der auf das Kolbenende des Spritzenkörpers aufgeschobenen Position entgegen der Aufschieberichtung ungewollt löst oder aus dieser Position entgegen der Aufschieberichtung abgezogen werden kann. Der Verbindungsmechanismus ist dabei insbesondere ausgebildet, das Abziehen des Mutterteils vom Spritzenkörper entgegen der Aufschieberichtung durch einen Benutzer mit leichtem bis normalem Kraftaufwand zu verhindern oder zu erschweren. Der erfindungsgemäße Verbindungsmechanismus muss nicht so ausgebildet sein, dass ein Abziehen entgegen der Aufschieberichtung auch bei erheblichem Kraftaufwand unmöglich ist. Insbesondere ist es bevorzugt, dass der Verbindungsmechanismus das Mutterteil auf dem Kolbenende in der Endposition des Mutterteils, die durch die Anschlagflächen definiert ist, fixiert und ein Abziehen aus dieser Endposition verhindert oder erschwert.

In einer bevorzugten Ausführungsform umfasst der Verbindungsmechanismus einen am Mutterteil ausgebildeten Flansch, der ein axiales Rastelement aufweist, das mit dem Kolbenende in Eingriff steht.

Der am Mutterteil ausgebildete Flansch mit dem axialen Rastelement kann mit dem Flansch des Mutterteils, der mit einer Gegenfläche am Kolbenende einen Formschluss bildet, identisch sein oder als separater, zweiter Flansch, vorzugsweise an einem dem vorgenannten Flansch gegenüberliegenden Ende des Innengewindes des Mutterteils, ausgebildet sein.

Ein axiales Rastelement ist zu verstehen als ein Rastelement, das eine Erstreckung in axialer Richtung aufweist und dazu dient, eine Rast- oder Schnappverbindung zwischen dem Mutterteil und dem Kolbenende beim Aufschieben des Mutterteils herzustellen. Insbesondere ist es bevorzugt, dass die Rast- oder Schnappverbindung zwischen Mutterteil und Kolbenende in der Endposition des Mutterteils, die durch die Anschlagflächen definiert ist, hergestellt wird.

In einer möglichen Fortbildungsform ist das Rastelement als Vorsprung vorzugsweise als Keil ausgebildet.

Durch diese Ausgestaltung des Rastelements kann beim Aufschieben des Mutterteils auf das Kolbenende das Kolbenende über den Vorsprung bzw. Keil geschoben werden und - vorzugsweise in der durch den Anschlag definierten Endposition des Mutterteils - hinter den Vorsprung bzw. Keil eingreifen bzw. einrasten oder einschnappen. Der Vorsprung bzw. der Keil hat dabei in axialer Richtung eine maximale Erstreckung, die derart dimensioniert ist, dass beim Aufschieben des Mutterteils auf das Kolbenende in radialer Aufschieberichtung das Kolbenende über den axialen Vorsprung gleitet und hinter dem axialen Vorsprung einrastet bzw. einschnappt.

In einer bevorzugten Ausführungsform ist ein Wandabschnitt der Hülse am Kolbenende axial zurückversetzt, wobei dieser Wandabschnitt mit dem Rastelement des Mutterteils in Eingriff steht.

Das Ausmaß, um das der Wandabschnitt der Hülse am Kolbenende axial zurückversetzt ist, ist vorzugsweise auf die axiale Erstreckung des Rastelements des Mutterteils, insbesondere eines als Vorsprung ausgebildeten Rastelements, abgestimmt. Diese Ausführungsform hat unter anderem den Vorteil, dass der axial zurückersetzte Wandabschnitt der Hülse dem Eingriff mit dem Rastelement des Mutterteils dienen kann, während der übrige Wandabschnitt der Hülse oder zumindest ein Teil davon beispielsweise als Gegenfläche am Kolbenende mit einem Flansch des Mutterteils einen Formschluss bilden kann, um eine Axialverschiebung des Mutterteils gegenüber dem Spritzenkörper in Austrittsrichtung zu verhindern.

In einer bevorzugten Ausführungsform weisen das Kolbenende und das Mutterteil jeweils eine Abdeckblende auf, wobei die Abdeckblenden derart ausgebildet und angeordnet sind, dass sie zusammen eine bis auf eine Gewindeöffnung des Mutterteils im Wesentlichen geschlossene Umhüllung des Kolbenendes und des Mutterteils bilden.

Durch de Abdeckung des Befestigungsmechanismus von Kolbenende und Mutterteil durch nach dem Aufschieben des Mutterteils auf das Kolbenende eine im Wesentlichen geschlossene Umhüllung bildende Abdeckblenden wird die Anfälligkeit der Drehspritze für Verunreinigungen deutlich reduziert. Die Abdeckblenden weisen vorzugsweise im Wesentlichen glatte Oberflächen auf und ein Spalt zwischen den beiden Abdeckblenden ist vorzugsweise einerseits so gering als möglich ausgebildet und andererseits so angeordnet, dass ein Anwender bei normalem Gebrauch die Spritze nicht vordringlich im Bereich des Spalts zwischen den Abdeckblenden berührt. Dies ist insbesondere vorteilhaft in Anwendungsgebieten, in denen hohe Anforderungen an die Hygiene der verwendeten Geräte und Werkzeuge gestellt werden, wie beispielsweise im Dentalbereich.

In einer möglichen Ausführungsform sind die Abdeckblenden im Wesentlichen auf in Umfangsrichtung gegenüberliegenden Hälften der Drehspritze angeordnet.

Bei dieser Ausführungsform befinden sich die Abdeckblenden im Wesentlichen auf gegenüberliegenden Seiten einer Trennebene, die die Längsachse umfasst. Dies ermöglicht auf einfache Weise eine ergonomische Gestaltung der Abdeckblenden zum Greifen durch einen Anwender.

In einer vorteilhaften Ausführungsform weisen die Abdeckblenden jeweils einen Abschnitt auf, der als Teil eines Hohlzylinderabschnitts ausgebildet ist und der zwischen zwei Greifflügeln angeordnet ist.

Die Ausbildung der Abdeckblenden mit jeweils einem Teil eines Hohlzylinderabschnitts dient einer Passung der Abdeckblenden zum vorzugsweise zumindest abschnittsweise hohlzylinderförmig ausgebildeten Kolbenende des Spritzenkörpers. Die Anordnung von zwei Greifflügeln auf beiden Seiten jedes Hohlzylinderabschnitts stellt eine ergonomische, vom Anwender gut zu greifende Ausgestaltung dar. Dies ist insbesondere bevorzugt, da der Anwender üblicherweise den Spritzenkörper mit dem aufgeschobenen Mutterteil in Bereich der Greifflügel der Abdeckblenden ergreifen und mit der anderen Hand die Kolbenspindel eindrehen wird.

In einer weiteren bevorzugten Ausführungsform sind die Anschlagflächen als Teil der Abdeckblenden ausgebildet.

Auf diese Weise ist sichergestellt, dass nach dem Aufschieben des Mutterteils auf das Kolbenende der Spalt zwischen den Abdeckblenden möglichst gering ist, da die Abdeckblenden als Anschlagflächen dienen und in der Endposition des Mutterteils auf dem Kolbenende miteinander in Eingriff stehen.

Die verbleibenden Spalte können darüber hinaus durch Klebemassen oder durch Verschweißen nahezu vollständig verschlossen werden.

In einer besonders bevorzugten Ausführungsform weisen das Kolbenende und das Mutterteil jeweils mindestens eine Anschlagfläche auf, die derart ausgebildet und angeordnet sind, dass sie in Aufschieberichtung einen Anschlag für das Mutterteil bilden, und das Kolbenende und/oder das Mutterteil einen Verbindungsmechanismus aufweist, der ein Abziehen des Mutterteils vom Kolbenende entgegen der Aufschieberichtung verhindert oder erschwert, und das Kolbenende und das Mutterteil jeweils eine Abdeckblende aufweisen, wobei die Abdeckblenden derart ausgebildet und angeordnet sind, dass sie zusammen eine bis auf eine Gewindeöffnung des Mutterteils im Wesentlichen geschlossene Umhüllung des Kolbenendes und des Mutterteils bilden.

In einer weiteren vorteilhaften Ausführungsform umfasst die Drehspritze eine im Spritzenkörper angeordnete Kolbenspitze umfassend einen ersten Abschnitt, dessen Außendurchmesser an einen Innendurchmesser der Hülse angepasst ist und der vorzugsweise eine Vertiefung für einen Eingriff mit einem Ende der Kolbenspindel aufweist, wobei die Kolbenspitze vorzugsweise ferner einen kegelstumpfförmigen Abschnitt umfasst, der an seinem zulaufenden Ende einen Außendurchmesser aufweist, der kleiner ist als der Innendurchmesser einer Austrittsöffnung am Austrittsende.

Der Außendurchmesser des ersten Abschnitts der Kolbenspitze kann je nach Materialeingenschaften von Kolbenspitze und Hülse geringfügig größer als oder gleich dem Innendurchmesser der Hülse ausgebildet sein, beispielsweise wenn als Materialien elastisch nachgebende Kunststoffe zum Einsatz kommen. Alternativ kann der Außendurchmesser des ersten Abschnitts der Kolbenspitze geringfügig kleiner als der Innendurchmesser der Hülse ausgebildet sein.

Die im Spritzenkörper angeordnete Kolbenspitze dient dazu, einen möglichen radialen Abstand zwischen der Kolbenspindel und der Innenwandung der Hülse - bedingt durch einen Außendurchmesser der Kolbenspindel, der kleiner ist als der Innendurchmesser der Hülse - auszugleichen, sodass beim Eindrehen der Kolbenspindel in der Hülse befindlicher Werkstoff über den gesamten Querschnittsbereich der Hülse zum Austrittsende hin vorgetrieben wird. Ein kegelstumpfförmiger Abschnitt der Kolbenspitze, der vorzugsweise zur Austrittsöffnung hin zuläuft, ist insbesondere dann vorteilhaft, wenn die Hülse des Spritzenkörpers am Austrittsende kegelstumpfförmig zuläuft, da auf diese Weise ein Austreiben des Werkstoffs erzielt werden kann, bei dem möglichst wenig Werkstoff im Spritzenkörper zurückbleibt, sodass ein vollständiges oder nahezu vollständiges Austreiben des Werkstoffs aus dem Spritzenkörper durch Betätigen der Spindel möglich ist.

Die erfindungsgemäße Drehspritze wird vorzugsweise nach einem Verfahren zur Abgabe eines Werkstoffs, insbesondere einer pastösen und/oder fließfähigen Dentalsubstanz, betätigt, umfassend die Schritte: Bereitstellen einer Drehspritze nach einer der vorgenannten Ausführungsformen und Eindrehen der Kolbenspindel in das Innengewinde des auf das Kolbenende aufgeschobenen Mutterteils.

Gemäß eines weiteren Aspekts der Erfindung wird die Aufgabe gelöst durch einen Spritzenkörper für eine Drehspritze nach Anspruch 13.

Die Gegenhalteflächen sind derart gegenüber der Längsachse geneigt, dass eine radiale, senkrecht zur Aufschieberichtung gerichtete Relativbewegung zwischen entsprechend geneigten Halteflächen und den Gegenhalteflächen verhindert ist.

Eine bevorzugte Ausführungsform des Spritzenkörpers ist dadurch gekennzeichnet, dass die Gegenhalteflächen derart gegenüber der Längsachse hinterschnitten sind, dass eine radiale, senkrecht zur Aufschieberichtung gerichtete Relativbewegung zwischen entsprechend hinterschnittenen Halteflächen und den Gegenhalteflächen verhindert ist.

Eine bevorzugte Ausführungsform des Spritzenkörpers ist dadurch gekennzeichnet, dass die Gegenhalteflächen derart stufenförmig ausgebildet sind, dass eine radiale, senkrecht zur Aufschieberichtung gerichtete Relativbewegung zwischen entsprechend stufenförmig ausgebildeten Halteflächen und den Gegenhalteflächen verhindert ist.

Die Gegenhalteflächen sind mit den Halteflächen derart in einen Formschluss bringbar, dass ein durch eine bei Betätigung einer Kolbenspindel aufgebrachte Kraft verursachtes Aufspreizen eines Mutterteils oder Teilen davon nach außen verhindert ist.

Eine weitere bevorzugte Ausführungsform des Spritzenkörpers ist dadurch gekennzeichnet, dass die Gegenhalteflächen mit den Halteflächen derart in einen Formschluss bringbar sind, dass bei Aufbringen einer Axialkraft durch eine Betätigung einer Kolbenspindel an den Gegenhalteflächen Kraftkomponenten in einer Richtung wirken, die in einer zur Aufschieberichtung im Wesentlichen senkrechten Ebene liegen.

Eine weitere bevorzugte Ausführungsform des Spritzenkörpers ist dadurch gekennzeichnet, dass das Kolbenende mindestens eine Anschlagfläche aufweist, die derart ausgebildet und angeordnet ist, dass sie in Aufschieberichtung mit mindestens einer an einem Mutterteil ausgebildeten Anschlagfläche einen Anschlag für das Mutterteil bildet.

Eine weitere bevorzugte Ausführungsform des Spritzenkörpers ist dadurch gekennzeichnet, dass das Kolbenende einen Verbindungsmechanismus aufweist, der ein Abziehen eines Mutterteils vom Kolbenende entgegen der Aufschieberichtung verhindert oder erschwert.

Eine weitere bevorzugte Ausführungsform des Spritzenkörpers ist dadurch gekennzeichnet, dass das Kolbenende ausgebildet ist, mit einem an einem Mutterteil ausgebildeten axialen Rastelement in Eingriff zu stehen.

Eine weitere bevorzugte Ausführungsform des Spritzenkörpers ist dadurch gekennzeichnet, dass das Kolbenende ausgebildet ist, mit einem als Vorsprung, vorzugsweise als Keil, ausgebildeten Rastelement eines Mutterteils in Eingriff zu stehen.

Eine alternative bevorzugte Ausführungsform des Spritzenkörpers ist dadurch gekennzeichnet, dass das Kolbenende des Spritzenkörpers einen Vorsprung aufweist, der ausgebildet ist, mit einem als Vertiefung an einem Mutterteil ausgebildeten Rastelement in Eingriff zu stehen.

Eine weitere bevorzugte Ausführungsform des Spritzenkörpers ist dadurch gekennzeichnet, dass ein Wandabschnitt der Hülse am Kolbenende axial zurückversetzt ist, wobei dieser Wandabschnitt ausgebildet ist, mit einem axialen Rastelement eines Mutterteils in Eingriff zu stehen.

Eine weitere bevorzugte Ausführungsform des Spritzenkörpers ist dadurch gekennzeichnet, dass das Kolbenende eine Abdeckblende aufweist, wobei die Abdeckblende derart ausgebildet und angeordnet sind, dass sie zusammen mit einer an einem Mutterteil ausgebildeten Abdeckblende eine bis auf eine Gewindeöffnung des Mutterteils im Wesentlichen geschlossene Umhüllung des Kolbenendes und des Mutterteils bildet.

Eine weitere bevorzugte Ausführungsform des Spritzenkörpers ist dadurch gekennzeichnet, dass die Abdeckblende im Wesentlichen auf einer auf die Umfangsrichtung bezogenen Hälfte einer Drehspritze angeordnet ist.

Eine weitere bevorzugte Ausführungsform des Spritzenkörpers ist dadurch gekennzeichnet, dass die Abdeckblende einen Abschnitt aufweist, der als Teil eines Hohlzylinderabschnitts ausgebildet ist und der zwischen zwei Greifflügeln angeordnet ist.

Eine weitere bevorzugte Ausführungsform des Spritzenkörpers ist dadurch gekennzeichnet, dass die Anschlagfläche als Teil der Abdeckblende ausgebildet ist.

Der Spritzenkörper und seine bevorzugten Ausführungsformen weisen Merkmale auf, die sie insbesondere dafür geeignet machen, für eine erfindungsgemäße Drehspritze und ihre Fortbildungen verwendet zu werden. Zu den Ausführungsformen, spezifischen Merkmalen, Varianten und Vorteilen der Merkmale des Spritzenkörpers und seiner Fortbildungen wird auf die vorangegangene Beschreibung zu den entsprechenden Merkmalen der Drehspritze verwiesen.

Gemäß eines weiteren Aspekts der Erfindung wird die Aufgabe gelöst durch ein Mutterteil für eine Drehspritze nach Anspruch 14.

Die Halteflächen sind derart gegenüber der Längsachse geneigt sind, dass eine radiale, senkrecht zur Aufschieberichtung gerichtete Relativbewegung zwischen den Halteflächen und entsprechend geneigten Gegenhalteflächen verhindert ist.

Eine bevorzugte Ausführungsform des Mutterteils ist dadurch gekennzeichnet, dass die Halteflächen derart gegenüber der Längsachse hinterschnitten sind, dass eine radiale, senkrecht zur Aufschieberichtung gerichtete Relativbewegung zwischen den Halteflächen und entsprechend hinterschnittenen Gegenhalteflächen verhindert ist.

Eine bevorzugte Ausführungsform des Mutterteils ist dadurch gekennzeichnet, dass die Halteflächen derart stufenförmig ausgebildet sind, dass eine radiale, senkrecht zur Aufschieberichtung gerichtete Relativbewegung zwischen den Halteflächen und entsprechend stufenförmig ausgebildeten Gegenhalteflächen verhindert ist

Die Halteflächen sind mit den Gegenhalteflächen derart in einen Formschluss bringbar sind, dass ein durch eine bei Betätigung einer Kolbenspindel aufgebrachte Kraft verursachtes Aufspreizen des Mutterteils oder Teilen davon nach außen verhindert ist.

Eine weitere bevorzugte Ausführungsform des Mutterteils ist dadurch gekennzeichnet, dass die Halteflächen mit den Gegenhalteflächen derart in einen Formschluss bringbar sind, dass bei Aufbringen einer Axialkraft durch eine Betätigung einer Kolbenspindel an den Halteflächen Kraftkomponenten in einer Richtung wirken, die in einer zur Aufschieberichtung im Wesentlichen senkrechten Ebene liegen.

Eine weitere bevorzugte Ausführungsform des Mutterteils ist dadurch gekennzeichnet, dass das Kolbenende mindestens eine Anschlagfläche aufweist, die derart ausgebildet und angeordnet ist, dass sie mit einer an einem Kolbenende eines Spritzenköpers ausgebildeten Anschlagfläche in Aufschieberichtung einen Anschlag für das Mutterteil bildet.

Eine weitere bevorzugte Ausführungsform des Mutterteils ist dadurch gekennzeichnet, dass das Mutterteil einen Verbindungsmechanismus aufweist, der ein Abziehen des Mutterteils von einem Kolbenende eines Spritzenkörpers entgegen der Aufschieberichtung verhindert oder erschwert.

Eine bevorzugte Ausführungsform des Mutterteils ist dadurch gekennzeichnet, dass der Verbindungsmechanismus einen am Mutterteil ausgebildeten Flansch umfasst, der ein axiales Rastelement aufweist, das ausgebildet ist, mit einem Kolbenende eines Spritzenkörpers in Eingriff zu stehen.

Eine weitere bevorzugte Ausführungsform des Mutterteils ist dadurch gekennzeichnet, dass das Rastelement als Vorsprung, vorzugsweise als Keil, ausgebildet ist.

Eine alternative bevorzugte Ausführungsform des Mutterteils ist dadurch gekennzeichnet, dass das Rastelement eine Vertiefung aufweist, die ausgebildet ist, mit einem an einem Kolbenende eines Spritzenkörpers ausgebildeten Vorsprung in Eingriff zu stehen.

Eine weitere bevorzugte Ausführungsform des Mutterteils ist dadurch gekennzeichnet, dass das Rastelement ausgebildet ist, mit einem axial zurückversetzten Wandabschnitt einer Hülse an einem Kolbenende eines Spritzenkörpers in Eingriff zu stehen.

Eine weitere bevorzugte Ausführungsform des Mutterteils ist dadurch gekennzeichnet, dass das Mutterteil eine Abdeckblende aufweist, die ausgebildet und angeordnet ist, dass sie zusammen mit einer an einem Kolbenende eines Spritzenkörpers ausgebildeten Abdeckblende eine bis auf eine Gewindeöffnung des Mutterteils im Wesentlichen geschlossene Umhüllung des Kolbenendes und des Mutterteils bildet.

Eine weitere bevorzugte Ausführungsform des Mutterteils ist dadurch gekennzeichnet, dass die Abdeckblende im Wesentlichen auf einer auf die Umfangsrichtung bezogenen Hälfte einer Drehspritze angeordnet ist.

Eine weitere bevorzugte Ausführungsform des Mutterteils ist dadurch gekennzeichnet, dass die Abdeckblende einen Abschnitt aufweist, der als Teil eines Hohlzylinderabschnitts ausgebildet ist und der zwischen zwei Greifflügeln angeordnet ist.

Eine bevorzugte Ausführungsform des Mutterteils ist dadurch gekennzeichnet, dass die Anschlagfläche als Teil der Abdeckblende ausgebildet ist.

Das Mutterteil und seine bevorzugten Ausführungsformen weisen Merkmale auf, die sie insbesondere dafür geeignet machen, für eine erfindungsgemäße Drehspritze und ihre Fortbildungen verwendet zu werden. Zu den Ausführungsformen, spezifischen Merkmalen, Varianten und Vorteilen der Merkmale des Mutterteils und seiner Fortbildungen wird auf die vorangegangene Beschreibung zu den entsprechenden Merkmalen der Drehspritze verwiesen.

Eine bevorzugte Ausführungsform der Erfindung wird beispielhaft anhand der beiliegenden Figuren beschrieben. Es zeigen:
- Figur 1:: Eine dreidimensionale Ansicht einer beispielhaften Ausführungsform einer erfindungsgemäßen Drehspritze,
- Figur 2:: eine andere dreidimensionale Ansicht der Drehspritze gemäß Figur 1,
- Figur 3:: eine Draufsicht auf die Drehspritze gemäß Figur 1,
- Figur 4:: eine Unteransicht der Drehspritze gemäß Figur 1,
- Figur 5:: eine weitere dreidimensionale Ansicht der Drehspritze gemäß Figur 1,
- Figur 6:: eine weitere dreidimensionale Ansicht der Drehspritze gemäß Figur 1,
- Figur 7:: eine weitere dreidimensionale Ansicht der Drehspritze gemäß Figur 1,
- Figur 8:: eine Seitenansicht der Drehspritze gemäß Figur 1,
- Figur 9:: einen Längsschnitt entlang einer ersten Schnittebene durch die Drehspritze gemäß Figur 1,
- Figur 10:: einen Längsschnitt entlang einer zweiten Schnittebene durch die Drehspritze gemäß Figur 1,
- Figur 11:: einen vergrößerten Ausschnitt aus Figur 9 ohne Schraffur,
- Figur 12:: eine dreidimensionale Ansicht der Drehspritze gemäß Figur 1 mit noch nicht aufgeschobenem Mutterteil,
- Figur 13:: eine dreidimensionale Ansicht des Mutterteils für die Drehspritze gemäß Figur 1 und
- Figur 14:: einen vergrößerten und teilweise geschnittenen Ausschnitt aus Figur 5.

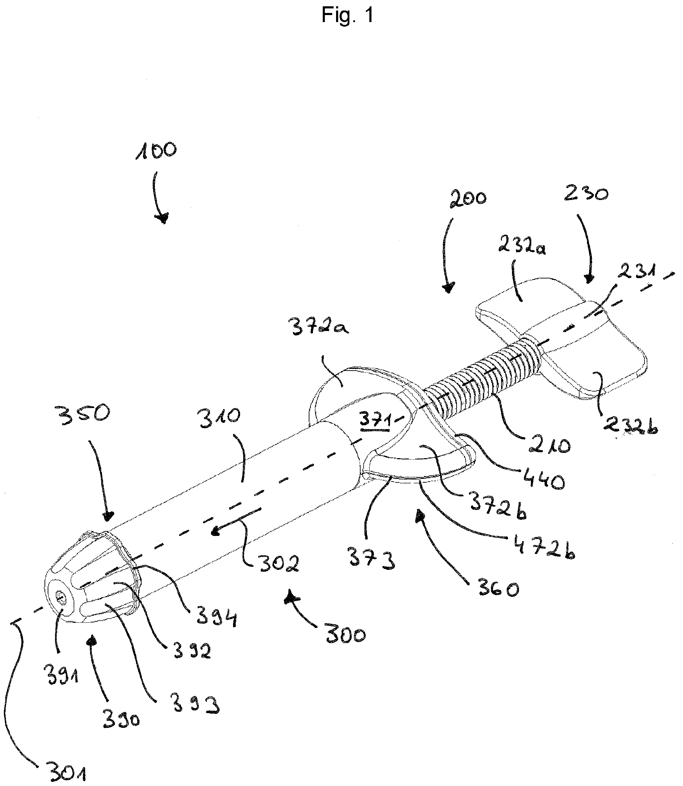

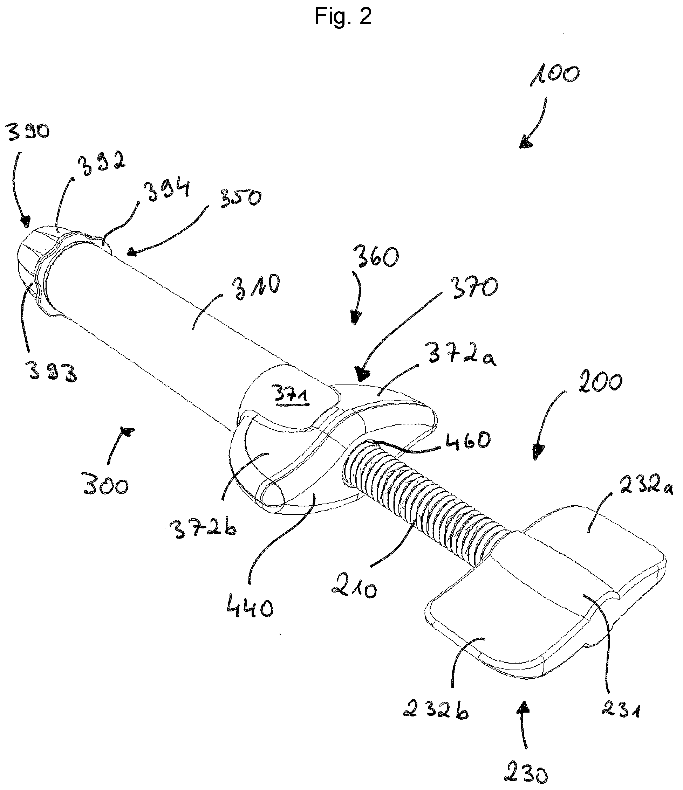

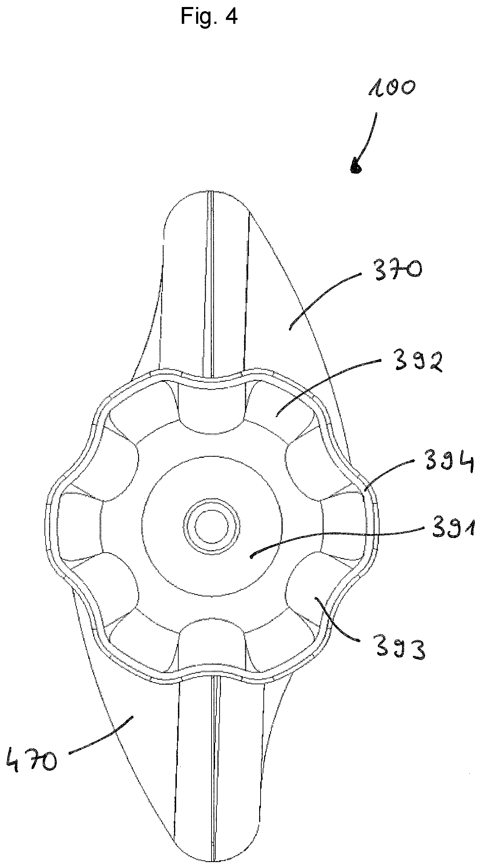

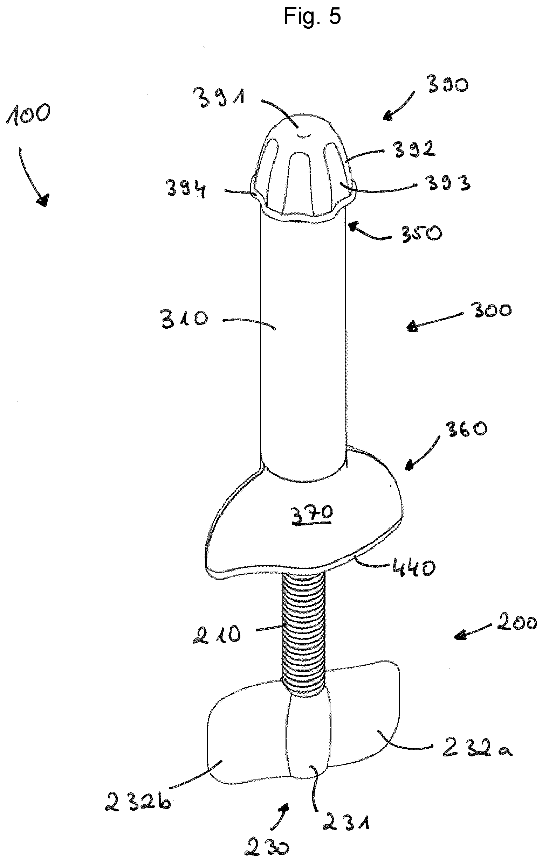

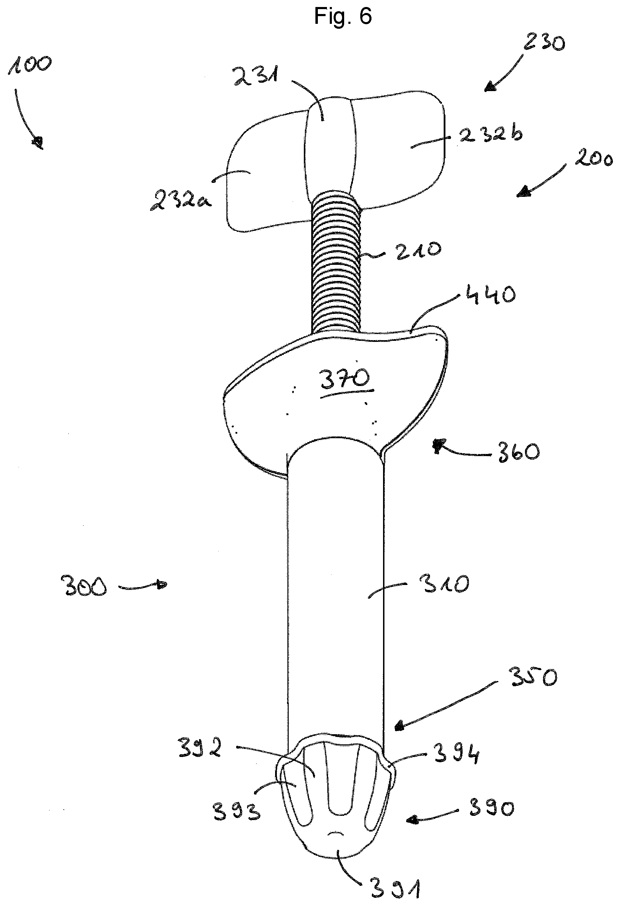

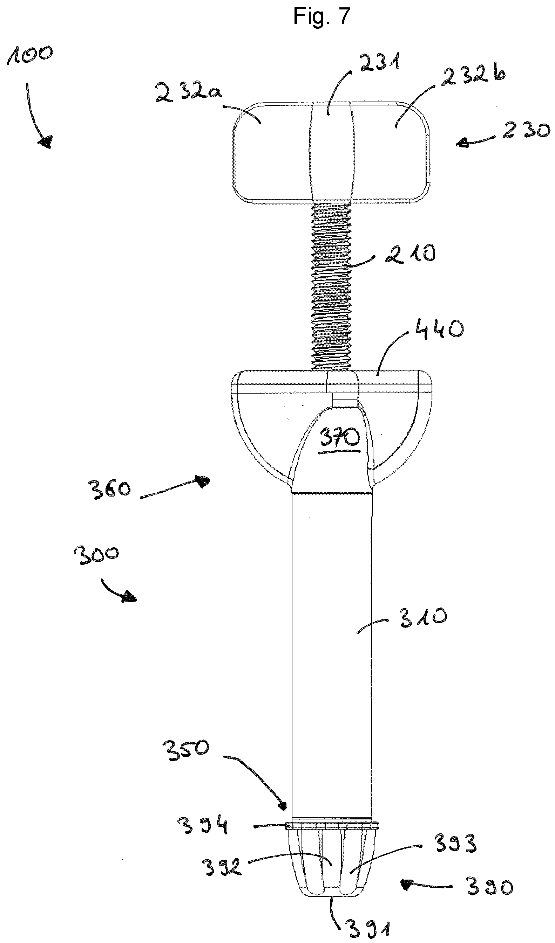

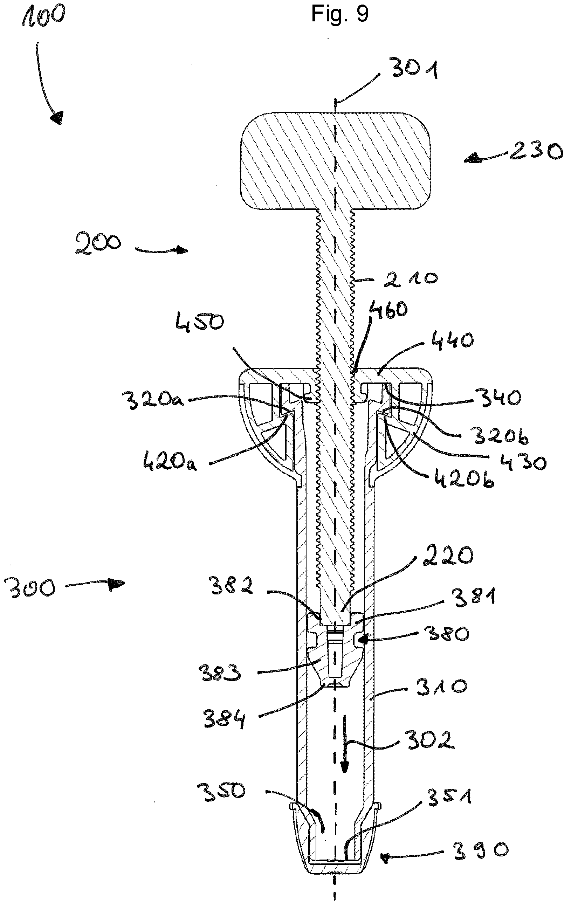

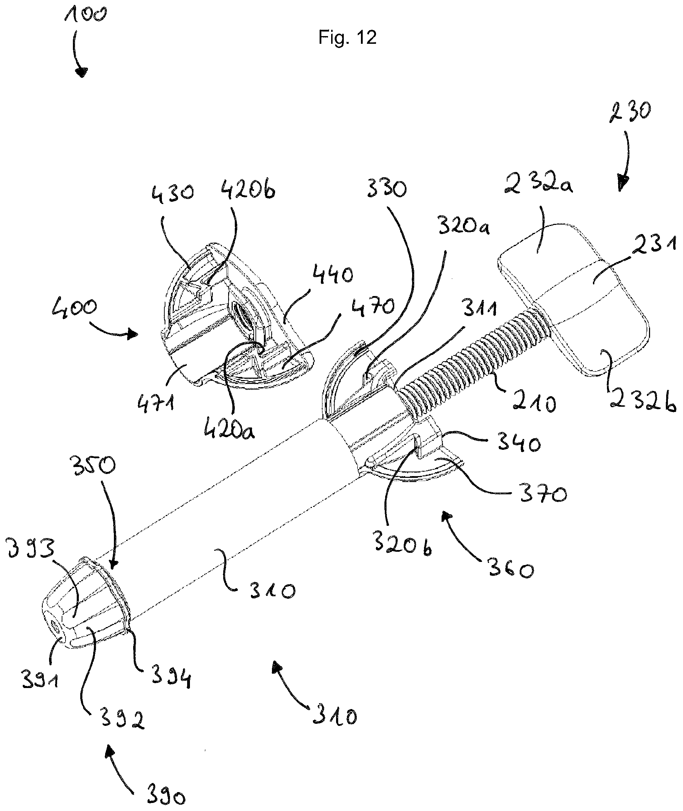

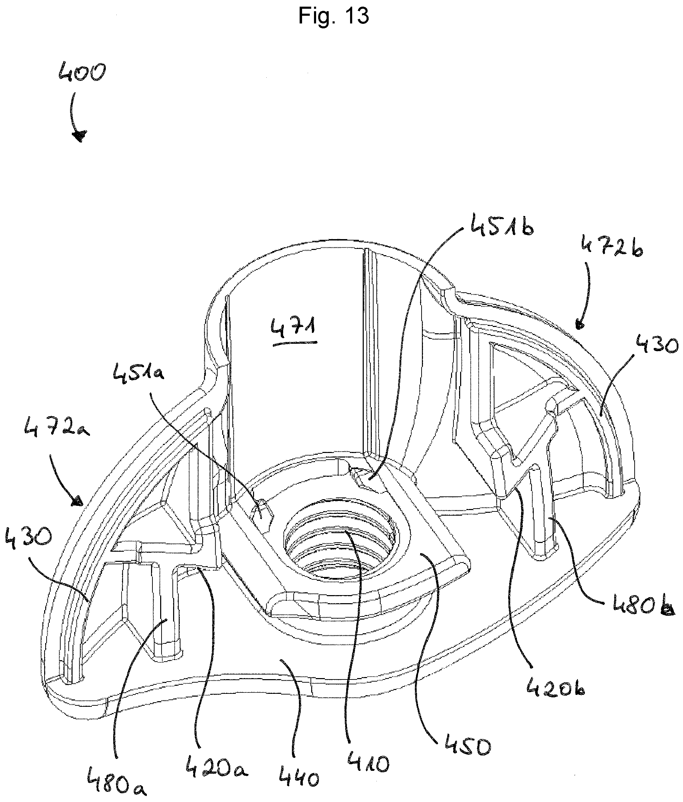

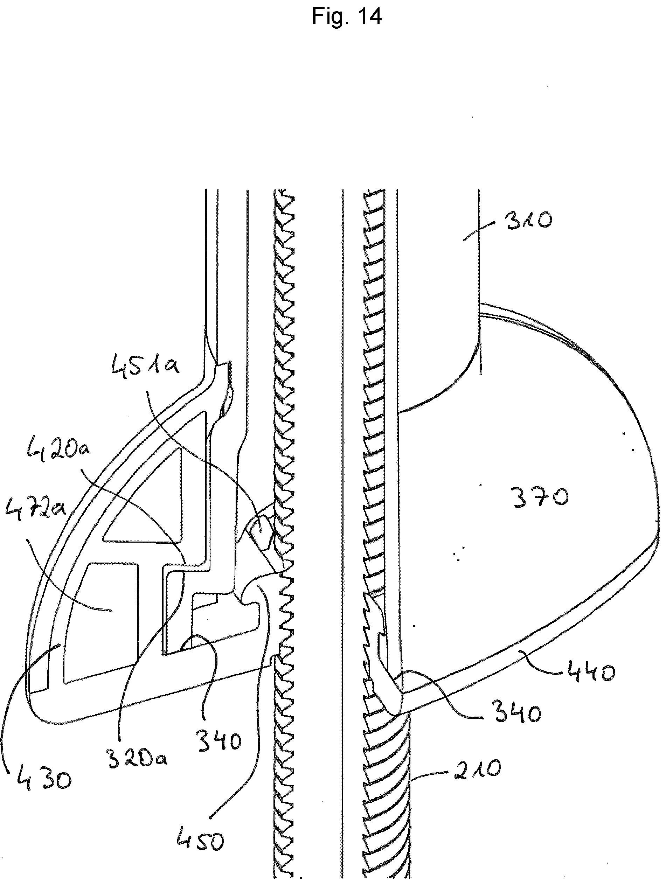

In den Figuren 1 bis 8 sind Ansichten einer beispielhaften Ausführungsform einer erfindungsgemäßen Drehspritze 100 zur Abgabe eines Werkstoffs (nicht dargestellt) insbesondere einer pastösen und/oder fließfähigen Dentalsubstanz, dargestellt. In den Figuren 9 bis 11 ist die Drehspritze 100 in Schnittansichten dargestellt und in Figur 12 in einer dreidimensionalen Ansicht mit abgenommenem Mutterteil 400, das in Figur 13 einzeln abgebildet ist. Figur 14 schließlich ist ein teilweise geschnittener Ausschnitt einer dreidimensionalen Darstellung der Drehspritze 100.

Die Drehspritze 100 umfasst eine Kolbenspindel 200 mit einem Außengewinde 210, einen Spritzenkörper 300 mit einer Längsachse 301, umfassend eine Hülse 310 zur Aufnahme eines Werkstoffs (nicht dargestellt), wobei die Hülse 310 ein Austrittsende 350 mit einer Austrittsöffnung 351 für den Austritt eines in der Hülse befindlichen Werkstoffs in einer axialen Austrittsrichtung 302 und ein Kolbenende 360 zur Aufnahme der Kolbenspindel 200 aufweist. Ferner umfasst die Drehspritze 100 ein Mutterteil 400 mit einem insbesondere in Figur 13 erkennbaren Innengewinde 410 für einen Eingriff mit dem Außengewinde 210 der Kolbenspindel 200. Die Drehspritze 100 weist ferner eine Kappe 390 auf, mit der die Austrittsöffnung 351 am Austrittsende 350 des Spritzenkörpers 300 verschlossen werden kann, was insbesondere im Lager- und Vertriebszustand der Spritze von Vorteil ist. Die Kappe 390 weist eine im Wesentlichen flache Kopfseite 391 sowie einen Kopfseite gegenüberliegenden ausgestellten Rand 394 auf sowie dazwischenliegende Rippen, die durch Vorwölbungen 392 und dazwischenliegende Vertiefungen 393 gebildet sind.

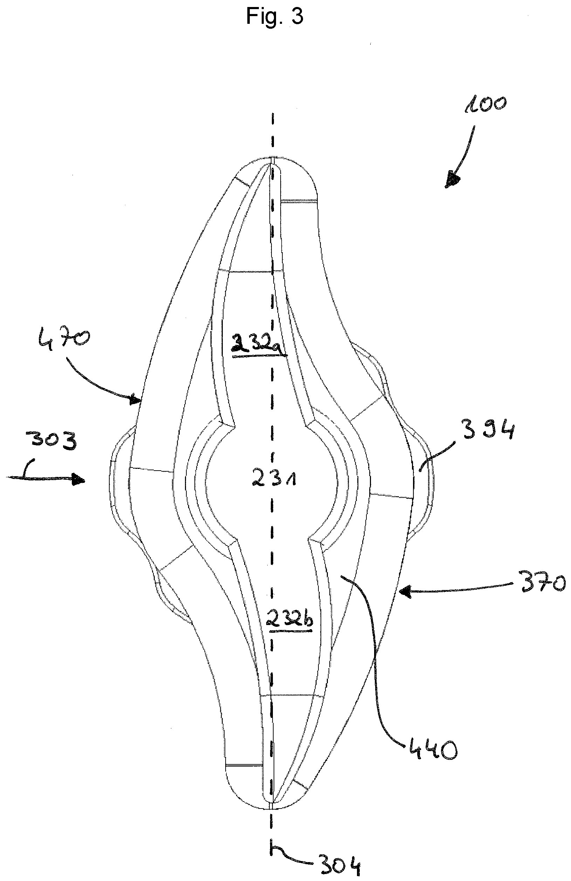

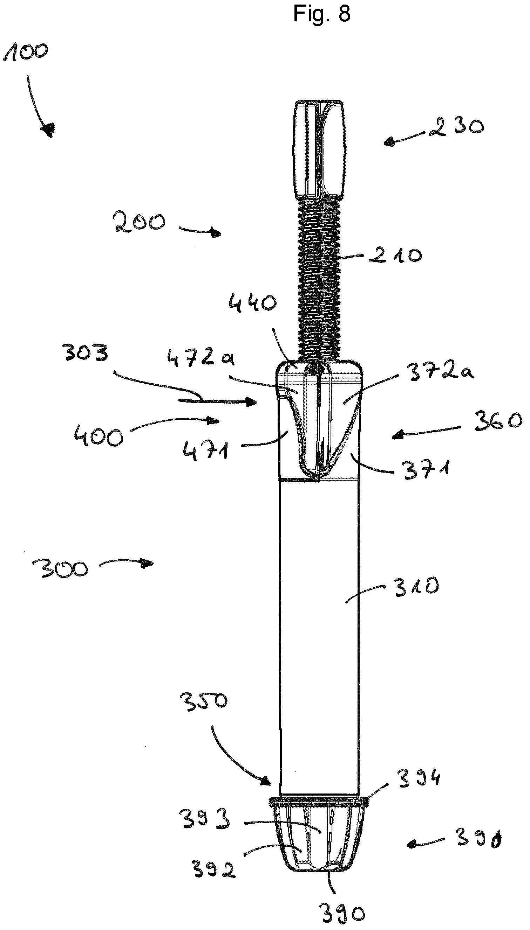

Das Mutterteil 400 ist in einer in den Figuren 3 und 8 mit den Pfeilen 303 bezeichneten radialen Aufschieberichtung auf das Kolbenende 360 des Spritzenkörpers 300 aufgeschoben.

Die Kolbenspindel weist ein Griffende 230 auf, das einen teilweise zylinderförmigen Abschnitt 231 aufweist, der zwischen zwei Greifflügeln 232a,b angeordnet ist. Dieser Greifabschnitt 230 ist derart ausgebildet, dass ein Anwender die Kolbenspindel 200 am Greifabschnitt 230 ergreifen und gegenüber dem Spritzenkörper 300 drehen kann, um die Kolbenspindel in der Hülse 310 vorzutreiben.

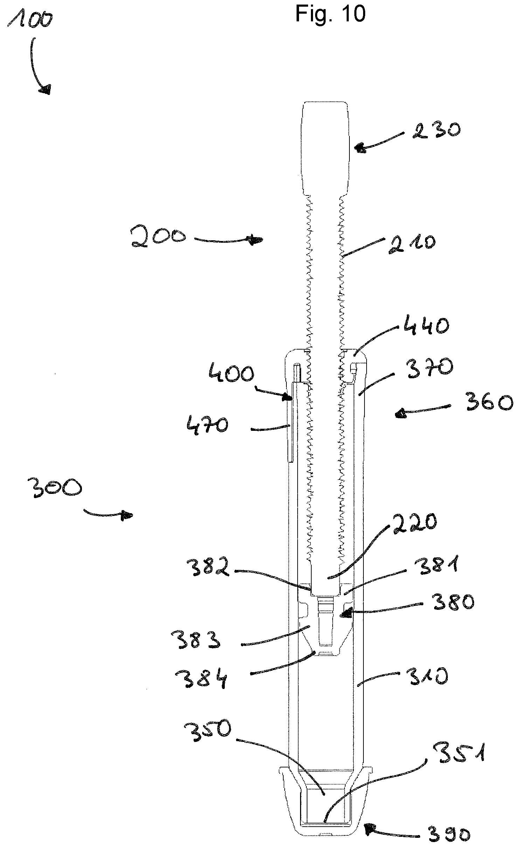

Wie in den Figuren 9 und 10 zu erkennen ist, weist die Drehspritze 100 ferner eine Kolbenspitze 380 auf. Die Kolbenspitze 380 umfasst einen ersten Abschnitt 381, dessen Außendurchmesser kleiner ist als ein Innendurchmesser der Hülse 310. Alternativ, insbesondere wenn die Kolbenspitze 380 und die Hülse 310 aus elastischem Kunststoff ausgebildet sind, kann der Außendurchmesser des ersten Abschnitts 381 der Kolbenspitze 380 auch gleich oder größer sein als der Innendurchmesser der Hülse 310. Der erste Abschnitt 381 weist ferner eine Vertiefung 382 für einen Eingriff mit einem Ende 220 der Kolbenspindel 200 auf. Das dem Greifende 230 gegenüberliegende Ende 220 der Kolbenspindel weist vorzugsweise kein Außengewinde auf. Die Kolbenspitze 380 weist ferner einen kegelstumpfförmigen Abschnitt 383 auf, der an seinem zulaufenden Ende 384 einen Außendurchmesser aufweist, der kleiner als der Innendurchmesser einer Austrittsöffnung 351 am Austrittsende 350 ist. Die Kolbenspitze 380 dient dazu, den Innenquerschnitt der Hülse 310 derart auszufüllen, dass mit der Kolbenspindel 210, deren Außendurchmesser vorzugsweise geringer ist als der Innendurchmesser der Hülse 310, in der Hülse 310 zwischen der Kolbenspitze 380 und der Austrittsöffnung 351 befindlicher Werkstoff durch Vortreiben der Kolbenspindel 200 und damit auch der Kolbenspitze 380 in der Hülse 310 durch die Austrittsöffnung 351 ausgetrieben werden kann.

Der Spritzenkörper 300 weist an seinem dem Austrittsende gegenüberliegenden Ende einen Bereich 360 auf, der als Kolbenende bezeichnet wird. Zur Herstellung der Drehspritze 100 wird das Mutterteil 400 in der radialen Aufschieberichtung 303 auf das Kolbenende 360 aufgedrückt.

In radialer Richtung bilden die Anschlagflächen 330 des Kolbenendes 360 und die Anschlagflächen 430 des Mutterteils 400 zusammen einen Anschlag, der eine Endposition des Mutterteils in Relation zu dem Spritzenkörper 300 definiert, wobei das Mutterteil 400 diese Endposition soweit auf das Kolbenende 360 aufgeschoben ist, dass die Anschlagflächen 430, 330 miteinander in Anschlag kommen und ein weiteres Aufschieben des Mutterteils auf das Kolbenende 360 nicht möglich ist. In dieser Endposition sind das Innengewinde 410 des Mutterteils 400 und die Hülse 310 des Spritzenkörpers 300 vorzugsweise koaxial ausgerichtet.

Als Anschlagfläche kann alternativ oder darüber hinaus der Abschnitt 471 zum Einsatz kommen.

Das Kolbenende 360 weist eine Abdeckblende 370 auf, an der die Anschlagfläche 330 des Kolbenendes 360 ausgebildet ist. Das Mutterteil 400 weist ebenfalls eine Abdeckblende 470 auf, an der die Anschlagfläche 430 ausgebildet ist. Die Abdeckblenden 370, 470 sind im Wesentlichen auf in Umfangsrichtung gegenüberliegenden Hälften der Drehspritze 100 angeordnet, dass heißt die Abdeckblende 370 befindet sich im Wesentlichen auf einer Abdeckblende 470 des Mutterteils gegenüberliegenden Hälfte einer Ebene 304, die die Längsachse 301 enthält und vorzugsweise senkrecht zur Aufschieberichtung 303 angeordnet ist, wie in insbesondere in Figur 3 zu erkennen ist. Die Abdeckblenden 370, 470 weisen jeweils einen Abschnitt 371, 471 auf, der als Teil eines Hohlzylinderabschnitts ausgebildet ist und der jeweils zwischen zwei Greifflügeln 372a,b, 472a,b angeordnet ist. Im gebrauchsfertigen Zustand der Drehspritze 100 mit auf das Kolbenende 360 aufgeschobenem Mutterteil 400 bilden die Abdeckflächen 370, 470 zusammen eine bis auf eine Gewindeöffnung 460 des Mutterteils 400 im Wesentlichen geschlossene Umhüllung des Kolbenendes 360 und des Mutterteils 400. Die Abdeckflächen 370, 470 sind dabei derart ausgebildet und angeordnet, dass ein Spalt 373 zwischen den Mutterteil 400 und dem Kolbenende 360 derart zwischen den Rändern der Greifflügel 372a,b, 472a,b angeordnet ist, dass beim Betätigen der Drehspritze und beim Ergreifen der Greifflügel durch einen Anwender der Spalt 373 nicht vordringlich berührt werden muss, sodass die Gefahr einer Verunreinigung der Drehspritze und insbesondere des Spalts 373 reduziert werden kann.

Darüber hinaus wird der Anwender die Anschlagflächen 430 des Mutterteils 400 durch das Greifen mit seinen Fingern gegen die Anschlagflächen 330 drücken.

Das Mutterteil 400 weist einen Flansch 440 auf, der nach dem Aufschieben des Mutterteils 400 auf das Kolbenende 360 mit der Gegenfläche 340 des Kolbenendes 360 derart einen Formschluss bildet, dass eine Axialverschiebung des Mutterteils 400 gegenüber dem Spritzenkörper 300 in Austrittsrichtung 302 verhindert ist. Das Mutterteil 400 weist ferner ein Paar Halteflächen 420a,b auf, die mit einem Paar Gegenhalteflächen 320a,b des Kolbenendes 360 nach dem Aufschieben des Mutterteils 400 auf das Kolbenende 360 derart einen Formschluss bilden, dass eine Axialverschiebung des Mutterteils 400 gegenüber dem Spritzenkörper 300 entgegen der Austrittsrichtung 302 verhindert ist. Auf diese Weise ist das Mutterteil 400 nach dem Aufschieben in axialer Richtung gegenüber dem Spritzenkörper 300 festgelegt. Die Halteflächen 420a,b des Mutterteils 400 können vorzugsweise an einem Paar von in axiale Richtung weisenden, vom Flansch 440 des Mutterteils abgehenden Armen 480a,b ausgebildet sein.

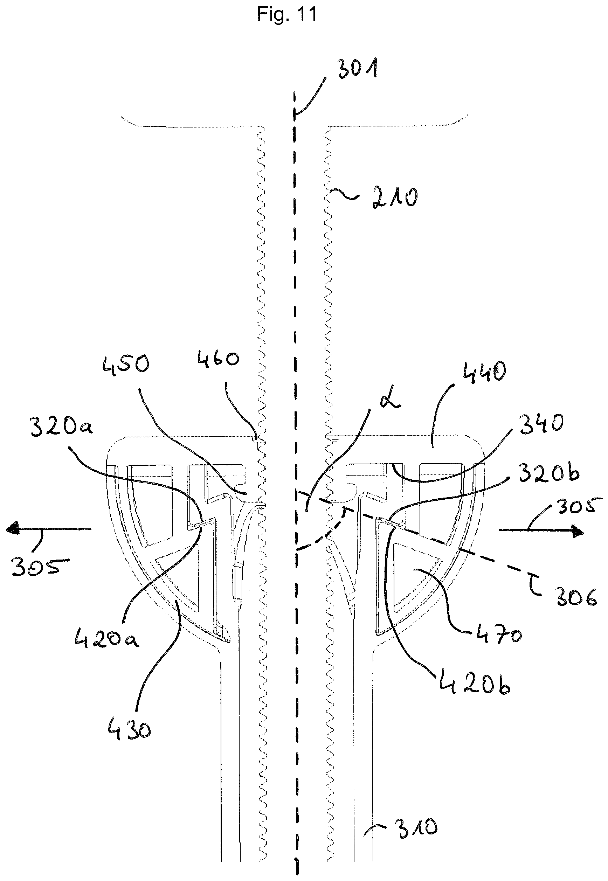

Die Halteflächen 420a,b des Mutterteils 400 und die Gegenhalteflächen 320a,b des Kolbenendes 360 stehen zudem derart einen Formschluss bilden, dass eine radiale, senkrecht zur Aufschieberichtung 303 gerichtete Relativbewegung zwischen den Halteflächen 420a,b und den Gegenhalteflächen 320a,b verhindert ist. In der hier dargestellten beispielshaften Ausführungsform ist der Formschluss durch einen durch eine Neigung der Halteflächen 420a,b und den Gegenhalteflächen 320a,b gegenüber der Längsachse 301 ausgebildeten Hinterschnitt realisiert. Die Halteflächen 420a,b und Gegenhalteflächen 320a,b sind in einem Winkel von 70° gegenüber der Längsachse 301 geneigt, was in Fig. 11 durch den Winkel alpha zwischen der mit 306 bezeichnete Ebene der Halteflächen 420b und der Gegenhalteflächen 320b und der Längsachse 306 erkennbar ist.

Auf diese Weise wird ein Aufspreizen des Mutterteils 400 beziehungsweise der Arme 480a,b mit den daran ausgebildeten Halteflächen 420a,b nach außen in Richtung der in Figur 11 mit 305 bezeichneten Pfeile verhindert, wenn bei Betätigung der Kolbenspindel eine Axialkraft aufgebracht wird. Die Halteflächen 420a,b und die Gegenhalteflächen 320a,b sind derart gegenüber der Längsachse 301 geneigt bzw. hinterschnitten, dass beim Aufbringen dieser Axialkraft durch eine Betätigung der Kolbenspindel 200 an den Halteflächen 420a,b und den Gegenhalteflächen 320a,b Kraftkomponenten in einer Richtung wirken, die in einer zur Aufschieberichtung 303 im Wesentlichen senkrechten Ebenen liegen. Insbesondere wirken diese Kraftkomponenten im Wesentlichen senkrecht zur Aufschieberichtung 303 und im Wesentlichen senkrecht zur Längsachse 301, sodass eine Bewegung der Halteflächen 420a,b des Mutterteils nach außen in Richtung der Pfeile 305 verhindert oder zumindest erschwert wird.

Das Kolbenende 360 und das Mutterteil 400 weisen ferner einen Verbindungsmechanismus auf, der ein Abziehen des Mutterteils 400 vom Kolbenende 360 entgegen der Aufschieberichtung 303 verhindert beziehungsweise zumindest soweit erschwert, dass bei bestimmungsgemäßer Handhabung der Drehspritze mit leichtem bis normalen Kraftaufwand eines Anwenders das Mutterteil 400 nicht vom Kolbenende 360 abgezogen werden kann. Der erfindungsgemäße Verbindungsmechanismus muss nicht verhindern, dass ein Abziehen mit stärkerem Kraftaufwand verhindert wird.

Der Verbindungsmechanismus umfasst einen am Mutterteil 400 ausgebildeten Flansch 450, der zwei axiale Rastelemente aufweist, die als keilförmige Vorsprünge 451 a,b ausgebildet sind. Diese keilförmigen Vorsprünge 451 a,b stehen in axialer Richtung über den Flansch 450 vor und sind aufgrund ihrer Ausdehnung in axialer Richtung auch als axiale Rastelemente bezeichnet. Alternativ (nicht dargestellt) kann ein Rastelement auch als eine in axiale Richtung weisende Vertiefung ausgebildet sein.

Wie insbesondere in Figur 12 zu erkennen ist, ist ein Wandabschnitt 311 der Hülse 310 am Kolbenende 360 axial zurückversetzt. Wie in Figur 12 weiter zu erkennen ist, ist der Wandabschnitt 311 als Teil eines Hohlzylinderabschnitts ausgebildet. Der axial zurückversetzte Wandabschnitt 311 gleitet beim Aufschieben des Mutterteils 400 auf das Kolbenende 360 über die als keilförmige Vorsprünge ausgebildeten axialen Rastelemente 451 a,b am Flansch 450 des Mutterteils 400 und rastet hinter den Rastelementen 451 a,b zur Herstellung einer Rast- beziehungsweise Schnappverbindung zwischen Mutterteil 400 und Kolbenende 360 ein, wenn das Mutterteil 400 durch Anschlag der Anschlagflächen 330, 430 seine Endposition erreicht hat. Auf diese Weise kann verhindert werden, dass das Mutterteil 400 vom Spritzenkörper 300 abfällt beziehungsweise leicht entgegen der Aufschieberichtung 302 abgezogen werden kann.

Der axial zurückversetzte Wandabschnitt 311 erlaubt darüber hinaus ein verhältnismäßig langes Innengewinde 410 im Mutterteil 400, ohne den Flansch 440 über seine gesamte Ausdehnung sehr dickwandig ausgestalten zu müssen und ohne einen in Richtung des Griffendes 230 der Spindel herausstehenden Fortsatz zu benötigen.

Der Außendurchmesser der Kolbenspindel 200, insbesondere des Außengewindes 210, und der Innendurchmesser der Hülse 310 beziehungsweise der Abschnitte 371, 471 der Abdeckblenden 370, 470 sowie des Wandabschnitts 311 sind dabei derart aufeinander abgestimmt, dass die Rastelemente 451 a,b im Zwischenraum zwischen der Kolbenspindel und der inneren Wandung der Hülse 310 beziehungsweise der Abschnitte 471, 371 der Abdeckflächen und des Wandabschnitts 311 angeordnet sein können, wie insbesondere in Figur 14 zu erkennen ist.

Spritzenkörper 300, Mutterteil 400, Kolbenspindel 200, Kolbenspitze 380 und Kappe 390 sind vorzugsweise im Spritzgussverfahren hergestellte, jeweils einstückige Kunststoffteile, wobei die Hülse 310 des Spritzenkörpers 300 mit dem abzugebenden Werkstoff befüllt wird. Die Teile Kolbenspindel 200, Spritzenkörper 300, Mutterteil 400, Kolbenspitze 380 und Kappe 390 werden zu einer gebrauchsfertigen Drehspritze 100 zusammengefügt. Dabei wird die Kolbenspitze 380 von Seiten des Kolbenendes 360 her in die Hülse eingeführt, das Mutterteil 400 in der radialen Aufschieberichtung 303 auf das Kolbenende 360 des Spritzenkörpers 300 aufgeschoben und die Kolbenspindel 200 mit ihrem Außengewinde 210 in das Innengewinde 410 des Mutterteils 400 eingedreht. Beim Aufschieben des Mutterteils 400 auf das Kolbenende 360 ist die Kolbenspindel 200 vorzugsweise noch nicht die Hülse 310 des Spritzenkörpers 300 eingeführt. Die Kappe 390 ist vorzugsweise auf das Austrittsende 350 lösbar aufgesteckt.

Zur Herstellung einer gebrauchsfertigen Drehspritze 100 gemäß der Erfindung ist insbesondere das Aufschieben des Mutterteils 400 auf das Kolbenende 360 des Spritzenkörpers 300 von Bedeutung. Dazu wird das in den Figuren 12 und 13 dargestellte Mutterteil 400 auf das Kolbenende 360 des Spritzenkörpers 300 in radialer Aufschieberichtung 303 aufgedrückt. Dabei wird das Mutterteil 400 so an das Kolbenende 360 angesetzt, dass zunächst die Kanten der Flansches 440 und 450 sowie der Halteflächen 420a,b mit den entsprechenden Kanten der Gegenfläche 340, des axial zurück versetzten Wandabschnitts 311 und der Gegenhalteflächen 320a,b in Berührung kommen. Beim weiteren Aufschieben bzw. Aufdrücken des Mutterteils 400 auf das Kolbenende 360 gleiten die jeweiligen Flächenpaarungen - Flansch 440 und Gegenfläche 340, Halteflächen 420a,b und Gegenhalteflächen 320a,b sowie Flansch 450 und Wandabschnitt 311 - aneinander entlang. Kurz vor Erreichen der Endposition des Mutterteils durch Anschlag der Anschlagflächen 330, 430, gleitet der axial zurückversetzte Wandabschnitt 311 über die als keilförmige Vorsprünge ausgebildeten Rastelemente 451 a,b des Flansches 450 des Mutterteils 400 und rastet in der Endposition hinter den keilförmigen Vorsprüngen 451 a,b ein. In dieser Endposition liegen die vorzugsweise etwa bogenförmig ausgestalteten Anschlagflächen 330, 430 vorzugsweise im Wesentlichen vollflächig aneinander an und der axial zurückversetzte Wandabschnitt 311 ist zwischen den axialen Rastelementen 451a,b und dem als Teil eines Hohlzylinders ausgebildeten Abschnitts 471 der Abdeckblende 470 angeordnet. In der Endposition liegen die Halteflächen 420a,b und Gegenhalteflächen 320a,b ebenfalls vorzugsweise im Wesentlichen vollflächig aneinander an. Die Gegenfläche 340 liegt auf der dem Flansch 450 zugewandten Seite des Flansches 440 des Mutterteils 400 an.

## Patentansprüche

1. Drehspritze (100) zur Abgabe eines Werkstoffs, insbesondere einer pastösen und/oder fließfähigen Dentalsubstanz, umfassend
- eine Kolbenspindel (200) mit einem Außengewinde (210),
- einen Spritzenkörper (300) mit einer Längsachse (301), umfassend eine Hülse (310) zur Aufnahme eines Werkstoffs, wobei die Hülse ein Austrittsende (350) für den Austritt eines in der Hülse befindlichen Werkstoffs in einer axialen Austrittsrichtung (302) und ein Kolbenende (360) zur Aufnahme der Kolbenspindel (200) aufweist, und
- ein Mutterteil (400) mit einem Innengewinde (410) für einen Eingriff mit dem Außengewinde (210) der Kolbenspindel (200),
wobei das Mutterteil (400) in einer radialen Aufschieberichtung (303) auf das Kolbenende (360) des Spritzenkörpers (300) aufgeschoben ist,
wobei
- das Mutterteil (400) einen Flansch (440) aufweist, der mit einer Gegenfläche (340) am Kolbenende (360) derart einen Formschluss bildet, dass eine Axialverschiebung des Mutterteils (400) gegenüber dem Spritzenkörper (300) in Austrittsrichtung (302) verhindert ist,
- das Mutterteil (400) Halteflächen (420a,b) aufweist, die mit Gegenhalteflächen (320a,b) des Kolbenendes (360) derart einen Formschluss bilden, dass eine Axialverschiebung des Mutterteils (400) gegenüber dem Spritzenkörper (300) entgegen der Austrittsrichtung (302) verhindert ist, und
- die Halteflächen (420a,b) und Gegenhalteflächen (320a,b) derart einen Formschluss bilden und gegenüber der Längsachse (301) geneigt sind,
- und einen Winkel mit der Längsachse (301) einschließen, der größer als 0° und kleiner als 90° ist,
- dass ein Aufspreizen des Mutterteils (400) oder Teilen davon nach außen, welches durch eine bei Betätigung der Kolbenspindel (200) aufgebrachte Kraft verursacht wird, verhindert ist und
- dass eine radiale, senkrecht zur Aufschieberichtung gerichtete Relativbewegung zwischen den Halteflächen und den Gegenhalteflächen verhindert ist.

2. Drehspritze (100) nach dem vorhergehenden Anspruch,
**dadurch gekennzeichnet, dass** die Halteflächen (420a,b) und Gegenhalteflächen (320a,b) derart hinterschnitten sind, dass eine radiale, senkrecht zur Aufschieberichtung (303) gerichtete Relativbewegung zwischen den Halteflächen (420a,b) und den Gegenhalteflächen (320a,b) verhindert ist.

3. Drehspritze (100) nach einem der vorhergehenden Ansprüche,
**dadurch gekennzeichnet, dass** die Halteflächen (420a,b) und Gegenhalteflächen (320a,b) derart stufenförmig ausgebildet sind, dass eine radiale, senkrecht zur Aufschieberichtung (303) gerichtete Relativbewegung zwischen den Halteflächen (420a,b) und den Gegenhalteflächen (320a,b) verhindert ist.

4. Drehspritze (100) nach einem der vorhergehenden Ansprüche,
**dadurch gekennzeichnet, dass** die Halteflächen (420a,b) und Gegenhalteflächen (320a,b) derart einen Formschluss bilden, dass bei Aufbringen einer Axialkraft durch eine Betätigung der Kolbenspindel (200) an den Halteflächen (420a,b) und Gegenhalteflächen (320a,b) Kraftkomponenten in einer Richtung wirken, die in einer zur Aufschieberichtung (303) im Wesentlichen senkrechten Ebene liegen.

5. Drehspritze (100) nach einem der vorhergehenden Ansprüche,
**dadurch gekennzeichnet, dass** das Kolbenende (360) und das Mutterteil (400) jeweils mindestens eine Anschlagfläche (330, 430) aufweisen, die derart ausgebildet und angeordnet sind, dass sie in Aufschieberichtung (303) einen Anschlag für das Mutterteil bilden.

6. Drehspritze (100) nach einem der vorhergehenden Ansprüche,
**dadurch gekennzeichnet, dass** das Kolbenende (360) und/oder das Mutterteil (400) einen Verbindungsmechanismus aufweist, der ein Abziehen des Mutterteils (400) vom Kolbenende (360) entgegen der Aufschieberichtung (303) verhindert oder erschwert.

7. Drehspritze (100) nach dem vorhergehenden Anspruch,
**dadurch gekennzeichnet, dass** der Verbindungsmechanismus einen am Mutterteil (400) ausgebildeten Flansch (450) umfasst, der ein axiales Rastelement (451a,b) aufweist, das mit dem Kolbenende (360) in Eingriff steht, wobei vorzugsweise das Rastelement (451a,b) als Vorsprung, vorzugsweise als Keil, ausgebildet ist oder das Rastelement eine Vertiefung aufweist, die mit einem am Kolbenende des Spritzenkörpers ausgebildeten Vorsprung in Eingriff steht.

8. Drehspritze (100) nach dem vorhergehenden Anspruch,
**dadurch gekennzeichnet, dass** ein Wandabschnitt (311) der Hülse (310) am Kolbenende (360) axial zurückversetzt ist, wobei dieser Wandabschnitt (311) mit dem Rastelement (451 a,b) des Mutterteils (400) in Eingriff steht.

9. Drehspritze (100) nach einem der vorhergehenden Ansprüche,
**dadurch gekennzeichnet, dass** das Kolbenende (360) und das Mutterteil (400) jeweils eine Abdeckblende (370, 470) aufweisen, wobei die Abdeckblenden (370, 470) derart ausgebildet und angeordnet sind, dass sie zusammen eine bis auf eine Gewindeöffnung (460) des Mutterteils (400) im Wesentlichen geschlossene Umhüllung des Kolbenendes (360) und des Mutterteils (400) bilden, wobei vorzugsweise die Abdeckblenden (370, 470) im Wesentlichen auf in Umfangsrichtung gegenüberliegenden Hälften der Drehspritze (100) angeordnet sind.

10. Drehspritze (100) nach dem vorhergehenden Anspruch,
**dadurch gekennzeichnet, dass** die Abdeckblenden (370, 470) jeweils einen Abschnitt aufweisen, der als Teil eines Hohlzylinderabschnitts (371, 471) ausgebildet ist und der zwischen zwei Greifflügeln (372a,b, 472a,b) angeordnet ist.

11. Drehspritze (100) nach einem der vorhergehenden Ansprüche 6 und 10, **dadurch gekennzeichnet, dass** die Anschlagflächen (330, 430) als Teil der Abdeckblenden (370, 470) ausgebildet sind.

12. Drehspritze (100) nach einem der vorhergehenden Ansprüche,
**gekennzeichnet durch** eine im Spritzenkörper (300) angeordnete Kolbenspitze (380) umfassend einen ersten Abschnitt (381), dessen Außendurchmesser an einen Innendurchmesser der Hülse (310) angepasst ist und der vorzugsweise eine Vertiefung (382) für einen Eingriff mit einem Ende (220) der Kolbenspindel (200) aufweist, wobei die Kolbenspitze (380) vorzugsweise ferner einen kegelstumpfförmigen Abschnitt (383) umfasst, der an seinem zulaufenden Ende (384) einen Außendurchmesser aufweist, der kleiner ist als ein Innendurchmesser einer Austrittsöffnung (351) am Austrittsende (350).

13. Spritzenkörper (300) für eine Drehspritze (100) zur Abgabe eines Werkstoffs nach einem der vorhergehenden Ansprüche, umfassend
eine Hülse (310) zur Aufnahme eines Werkstoffs, wobei die Hülse ein Austrittsende (350) für den Austritt eines in der Hülse (310) befindlichen Werkstoffs in einer axialen Austrittsrichtung (302) und ein Kolbenende (360) zur Aufnahme einer Kolbenspindel (200) aufweist, und wobei das Kolbenende (360) derart ausgebildet ist, dass ein Mutterteil (400) für eine Drehspritze (100) nach einem der vorhergehenden Ansprüche in eine radiale Aufschieberichtung (303) auf das Kolbenende (360) aufschiebbar ist,
wobei
- das Kolbenende (360) eine Gegenfläche (340) aufweist, die mit einem am Mutterteil (400) ausgebildeten Flansch (440) derart in einen Formschluss bringbar ist, dass eine relative Axialverschiebung zwischen dem Mutterteil (400) und dem Spritzenkörper (300) in Austrittsrichtung (302) verhindert ist,
- das Kolbenende (360) Gegenhalteflächen (320a,b) aufweist, die mit an einem Mutterteil (400) ausgebildeten Halteflächen (420a,b) derart in einen Formschluss bringbar sind, dass eine relative Axialverschiebung zwischen dem Mutterteil (400) und dem Spritzenkörper (300) entgegen der Austrittsrichtung (302) verhindert ist, und
- die Gegenhalteflächen (320a,b) mit den Halteflächen (420a,b) derart in einen Formschluss bringbar und derart gegenüber der Längsachse (301) geneigt sind,
- und einen Winkel mit der Längsachse (301) einschließen, der größer als 0° und kleiner als 90° ist,
- dass ein Aufspreizen des Mutterteils (400) oder Teilen davon nach außen, welches durch eine bei Betätigung der Kolbenspindel (200) aufgebrachte Kraft verursacht wird, verhindert ist und
- dass eine radiale, senkrecht zur Aufschieberichtung gerichtete Relativbewegung zwischen den entsprechend geneigten Halteflächen und den Gegenhalteflächen verhindert ist.

14. Mutterteil (400) für eine Drehspritze (100) zur Abgabe eines Werkstoffs nach einem der vorhergehenden Ansprüche 1 bis 12 umfassend ein Innengewinde (410) für einen Eingriff mit einem Außengewinde (210) einer Kolbenspindel (200), wobei das Mutterteil (400) derart ausgebildet ist, dass es in eine radiale Aufschieberichtung (303) auf ein Kolbenende (360) eines Spritzenkörpers (300) für eine Drehspritze (100) nach einem der vorhergehenden Ansprüche 1 bis 12 aufschiebbar ist,
wobei
- das Mutterteil (400) einen Flansch (440) aufweist, der mit einer an einem Kolbenende (360) eines Spritzenkörpers (300) ausgebildeten Gegenfläche (340) derart in einen Formschluss bringbar ist, dass eine relative Axialverschiebung zwischen dem Mutterteil (400) und dem Spritzenkörper (300) in Austrittsrichtung (302) verhindert ist,
- das Mutterteil (400) Halteflächen (420a,b) aufweist, die mit an einem Kolbenende (360) eines Spritzenkörpers (300) ausgebildeten Gegenhalteflächen (320a,b) derart in einen Formschluss bringbar sind, dass eine relative Axialverschiebung zwischen dem Mutterteil (400) und dem Spritzenkörper (300) entgegen der Austrittsrichtung (303) verhindert ist, und
- die Halteflächen (420a,b) mit den Gegenhalteflächen (320a,b) derart in einen Formschluss bringbar und derart gegenüber der Längsachse (301) geneigt sind,
- und einen Winkel mit der Längsachse (301) einschließen, der größer als 0° und kleiner als 90° ist,
- dass ein Aufspreizen des Mutterteils (400) oder Teilen davon nach außen, welches durch eine bei Betätigung der Kolbenspindel (200) aufgebrachte Kraft verursacht wird, verhindert ist und
- dass eine radiale, senkrecht zur Aufschieberichtung gerichtete Relativbewegung zwischen den Halteflächen und den entsprechend geneigten Gegenhalteflächen verhindert ist.

## Claims

1. Screw syringe (100) for dispensing a material, in particular a pasty and/or fluid dental substance, comprising
- a plunger spindle (200) having an external thread (210);
- a syringe barrel (300) having a longitudinal axis (301), comprising a sleeve (310) for receiving a material, wherein the sleeve has an outflow end (350) out of which a material contained in the sleeve flows in an axial outflow direction (302), and a plunger end (360) for receiving the plunger spindle (200), and
- a nut part (400) having an internal thread (410) for meshing with the external thread (210) of the plunger spindle (200),
wherein the nut part (400) is slid in a radial sliding direction (303) onto the plunger end (360) of the syringe barrel (300),
wherein
- the nut part (400) has a flange (440) which creates a form fit with a counter surface (340) on the plunger end (360) such that an axial displacement of the nut part (400) relative to the syringe barrel (300) in the outflow direction (302) is prevented,
- the nut part (400) has retaining surfaces (420a, b) which create a form fit with counter-retaining surfaces (320a, b) of the plunger end (360) such that an axial displacement of the nut part (400) relative to the syringe barrel (300) counter to the outflow direction (302) is prevented, and
- the retaining surfaces (420a, b) and counter-retaining surfaces (320a, b) thus create a form fit, are inclined in relation to the longitudinal axis (301),
- and enclose an angle of greater than 0° and less than 90° together with the longitudinal axis (301) such that
- spreading apart of the nut part (400) or parts thereof outwards due to a force applied by operating the plunger spindle (200) is prevented, and
- a radial relative movement, perpendicular to the sliding direction, between the retaining surfaces and the counter-retaining surfaces is prevented.

2. Screw syringe (100) according to the preceding claim, **characterised in that** the retaining surfaces (420a, b) and counter-retaining surfaces (320a, b) are undercut such that a radial relative movement, perpendicular to the sliding direction (303), between the retaining surfaces (420a, b) and the counter-retaining surfaces (320a, b) is prevented.

3. Screw syringe (100) according to any of the preceding claims, **characterised in that** the retaining surfaces (420a, b) and counter-retaining surfaces (320a, b) have a stepped shape such that a radial relative movement, perpendicular to the sliding direction (303), between the retaining surfaces (420a, b) and the counter-retaining surfaces (320a, b) is prevented.

4. Screw syringe (100) according to any of the preceding claims, **characterised in that** the retaining surfaces (420a, b) and counter-retaining surfaces (320a, b) create a form fit such that, when an axial force is applied to the retaining surfaces (420a, b) and counter-retaining surfaces (320a, b) by operating the plunger spindle (200), force components act in a direction in a plane substantially perpendicular to the sliding direction (303).

5. Screw syringe (100) according to any of the preceding claims, **characterised in that** the plunger end (360) and the nut part (400) each have at least one abutting surface (330, 430) that are designed and arranged in such a way that they form a stop for the nut part in the sliding direction (303).

6. Screw syringe (100) according to any of the preceding claims, **characterised in that** the plunger end (360) and/or the nut part (400) has a connection mechanism that prevents or impedes detachment of the nut part (400) from the plunger end (360) counter to the sliding direction (303).

7. Screw syringe (100) according to the preceding claim, **characterised in that** the connection mechanism comprises a flange (450) that is formed on the nut part (400) and has an axial latching element (451 a, b), that engages with the plunger end (360), the latching element (451 a, b) preferably being in the form of a protrusion, preferably a wedge, or the latching element having a recess that engages with a protrusion formed on the plunger end of the syringe barrel.

8. Screw syringe (100) according to the preceding claim, **characterised in that** a wall portion (311) of the sleeve (310) on the plunger end (360) is axially set back, this wall portion (311) engaging with the latching element (451 a, b) of the nut part (400).

9. Screw syringe (100) according to any of the preceding claims, **characterised in that** the plunger end (360) and the nut part (400) each have a cover plate (370, 470), the cover plates (370, 470) being designed and arranged in such a way that, with the exception of a threaded opening (460) in the nut part (400), together they form a substantially enclosed covering of the plunger end (360) and the nut part (400), the cover plates (370, 470) preferably being arranged substantially on opposing halves of the screw syringe (100) in the circumferential direction.

10. Screw syringe (100) according to the preceding claim, **characterised in that** the cover plates (370, 470) each have a portion that is formed as part of a hollow cylindrical portion (371, 471) and is arranged between two wing grips (372a, b, 472a, b).

11. Screw syringe (100) according to any of the preceding claims 6 and 10, **characterised in that** the abutting surfaces (330, 430) are part of the cover plates (370, 470).

12. Screw syringe (100) according to any of the preceding claims, **characterised by** a plunger tip (380) arranged in the syringe barrel (300) comprising a first portion (381), the external diameter of which is matched to an internal diameter of the sleeve (310) and which preferably has a recess (382) for engaging with an end (220) of the plunger spindle (200), the plunger tip (380) preferably also comprising a frustoconical portion (383) which, at the tapered end (384) thereof, has an external diameter that is smaller than an internal diameter of an outflow opening (351) at the outflow end (350).

13. Syringe barrel (300) for a screw syringe (100) for dispensing a material, according to any of the preceding claims, comprising
a sleeve (310) for receiving a material, wherein the sleeve has an outflow end (350) out of which a material contained in the sleeve (310) flows in an axial outflow direction (302) and a plunger end (360) for receiving a plunger spindle (200), and wherein the plunger end (360) is designed such that a nut part (400) for a screw syringe (100) according to any of the preceding claims can slide in a radial sliding direction (303) onto the plunger end (360), wherein
- the plunger end (360) has a counter surface (340) that can be brought into a form fit with a flange (440) formed on the nut part (400) such that a relative axial displacement between the nut part (400) and the syringe barrel (300) in the outflow direction (302) is prevented,
- the plunger end (360) has counter-retaining surfaces (320a, b), that can be brought into a form fit with retaining surfaces (420a, b) formed on a nut part (400) such that a relative axial displacement between the nut part (400) and the syringe barrel (300) counter to the outflow direction (302) is prevented, and
- the counter-retaining surfaces (320a, b) can be brought into a form fit with the retaining surfaces (420a, b), are inclined in relation to the longitudinal axis (301),
- and enclose an angle of greater than 0° and less than 90° together with the longitudinal axis (301) such that
- spreading apart of the nut part (400) or parts thereof outwards due to a force applied by operating the plunger spindle (200) is prevented, and
- a radial relative movement, perpendicular to the sliding direction, between the retaining surfaces inclined accordingly and the counter-retaining surfaces is prevented.

14. Nut part (400) for a screw syringe (100) for dispensing a material according to any of the preceding claims 1 to 12, comprising
an internal thread (410) for meshing with an external thread (210) of a plunger spindle (200), wherein the nut part (400) is designed such that it can be slid in a radial sliding direction (303) onto a plunger end (360) of a syringe barrel (300) for a screw syringe (100) according to any of the preceding claims 1 to 12,
wherein
- the nut part (400) has a flange (440) which can be brought into a form fit with a counter surface (340) formed on a plunger end (360) of a syringe barrel (300) such that a relative axial displacement between the nut part (400) and the syringe barrel (300) in the outflow direction (302) is prevented;
- the nut part (400) has retaining surfaces (420a, b) which can be brought into a form fit with counter-retaining surfaces (320a, b) formed on a plunger end (360) of a syringe barrel (300) such that a relative axial displacement between the nut part (400) and the syringe barrel (300) counter to the outflow direction (303) is prevented, and
- the retaining surfaces (420a, b) can be brought into a form fit with the counter-retaining surfaces (320a, b), are inclined in relation to the longitudinal axis (301),
- and enclose an angle of greater than 0° and less than 90° together with the longitudinal axis (301) such that
- spreading apart of the nut part (400) or parts thereof outwards due to a force applied by operating the plunger spindle (200) is prevented, and
- a radial relative movement, perpendicular to the sliding direction, between the retaining surfaces and the counter-retaining surfaces inclined accordingly is prevented.

## Revendications

1. Seringue à vis (100) servant à distribuer une matière, en particulier une substance dentaire pâteuse et/ou pouvant s'écouler, comprenant
- une tige de piston (200) pourvue d'un filetage extérieur (210),
- un corps de seringue (300) à axe longitudinal (301), comprenant une douille (310) servant à recevoir une matière, sachant que la douille présente une extrémité de sortie (350) pour la sortie d'une matière se trouvant dans la douille dans une direction de sortie (302) axiale et une extrémité de piston (360) servant à recevoir la tige de piston (200), et
- une partie écrou (400) pourvue d'un filetage intérieur (410) pour une prise avec le filetage extérieur (210) de la tige de piston (200),
sachant que la partie écrou (400) est enfilée, dans une direction d'enfilement (303) radiale, sur l'extrémité de piston (360) du corps de seringue (300),
sachant que
- la partie écrou (400) présente une bride (440), qui forme une complémentarité de forme avec une face complémentaire (340) au niveau de l'extrémité de piston (360) de telle manière qu'un déplacement par coulissement axial de la partie écrou (400) par rapport au corps de seringue (300) dans la direction de sortie (302) est empêché,
- la partie écrou (400) présente des faces de maintien (420a, b), qui forment une complémentarité de forme avec les faces de maintien complémentaires (320a, b) de l'extrémité de piston (360) de telle manière qu'un déplacement par coulissement axial de la partie écrou (400) par rapport au corps de seringue (300) dans le sens inverse à la direction de sortie (302) est empêché, et
- les faces de maintien (420a, b) et les faces de maintien complémentaires (320a, b) forment une complémentarité de forme et sont inclinées par rapport à l'axe longitudinal (301),
- et forment, avec l'axe longitudinal (301), un angle qui est supérieur à 0° et inférieur à 90°, de telle manière
- qu'un écartement de la partie écrou (400) ou de parties de cette dernière vers l'extérieur, lequel peut être provoqué par une force appliquée lors d'un actionnement de la tige de piston (200), est empêché, et
- qu'un déplacement relatif radial orienté de manière perpendiculaire par rapport à la direction d'enfilement entre les faces de maintien et les faces de maintien complémentaires est empêché.

2. Seringue à vis (100) selon la revendication précédente,
**caractérisée en ce que** les faces de maintien (420a, b) et les faces de maintien complémentaires (320a, b) sont contre-dépouillées de telle manière qu'un déplacement relatif radial orienté de manière perpendiculaire par rapport à la direction d'enfilement (303) entre les faces de maintien (420a, b) et les faces de maintien complémentaires (320a, b) est empêché.

3. Seringue à vis (100) selon l'une quelconque des revendications précédentes,
**caractérisée en ce que** les faces de maintien (420a, b) et les faces de maintien complémentaires (320a, b) sont réalisées de manière à présenter une forme étagée de telle manière qu'un déplacement relatif radial orienté de manière perpendiculaire par rapport à la direction d'enfilement (303) entre les faces de maintien (420a, b) et les faces de maintien complémentaires (320a, b) est empêché.

4. Seringue à vis (100) selon l'une quelconque des revendications précédentes,
**caractérisée en ce que** les faces de maintien (420a, b) et les faces de maintien complémentaires (320a, b) forment une complémentarité de forme de telle manière que, lors de l'application d'une force axiale par un actionnement de la tige de piston (200) au niveau des faces de maintien (420a, b) et des faces de maintien complémentaires (320a, b), des composantes de force qui se trouvent dans un plan sensiblement perpendiculaire par rapport à la direction d'enfilement (303) agissent dans une direction donnée.

5. Seringue à vis (100) selon l'une quelconque des revendications précédentes,
**caractérisée en ce que** l'extrémité de piston (360) et la partie écrou (400) présentent respectivement au moins une face de butée (330, 430), lesquelles faces de butée sont réalisées et disposées de telle manière qu'elles forment, dans une direction d'enfilement (303), une butée pour la partie écrou.

6. Seringue à vis (100) selon l'une quelconque des revendications précédentes,
**caractérisée en ce que** l'extrémité de piston (360) et/ou la partie écrou (400) présentent un mécanisme de liaison, qui empêche ou complique un retrait de la partie écrou (400) hors de l'extrémité de piston (360) dans le sens inverse à la direction d'enfilement (303).

7. Seringue à vis (100) selon la revendication précédente,
**caractérisée en ce que** le mécanisme de liaison comprend une bride (450) réalisée au niveau de la partie écrou (400), laquelle bride présente un élément d'enclenchement (451a, b) axial, qui est en prise avec l'extrémité de piston (360), sachant que de préférence l'élément d'enclenchement (451a, b) est réalisé sous la forme d'une partie faisant saillie, de préférence sous la forme d'une clavette, ou que l'élément d'enclenchement présente un renfoncement, qui est en prise avec une partie faisant saillie réalisée au niveau de l'extrémité de piston du corps de seringue.

8. Seringue à vis (100) selon la revendication précédente,
**caractérisée en ce qu'**une section de paroi (311) de la douille (310) est décalée en arrière de manière axiale au niveau de l'extrémité de piston (360), sachant que ladite section de paroi (311) est en prise avec l'élément d'enclenchement (451a, b) de la partie écrou (400).

9. Seringue à vis (100) selon l'une quelconque des revendications précédentes,
**caractérisée en ce que** l'extrémité de piston (360) et la partie écrou (400) présentent respectivement un cache de recouvrement (370, 470), sachant que les caches de recouvrement (370, 470) sont réalisés et disposés de telle manière qu'ils forment ensemble une enveloppe sensiblement fermée, allant jusqu'à une ouverture filetée (460) de la partie écrou (400), de l'extrémité de piston (360) et de la partie écrou (400), sachant que de préférence les caches de recouvrement (370, 470) sont disposés sensiblement sur des moitiés, se faisant face dans la direction périphérique, de la seringue à vis (100).

10. Seringue à vis (100) selon la revendication précédente,
**caractérisée en ce que** les caches de recouvrement (370, 470) présentent respectivement une section, qui est réalisée sous la forme d'une partie d'une section cylindrique creuse (371, 471) et qui est disposée entre deux ailettes de préhension (372a, b, 472a, b).

11. Seringue à vis (100) selon l'une quelconque des revendications 6 et 10,
**caractérisée en ce que** les faces de butée (330, 430) sont réalisées en tant que partie intégrante des caches de recouvrement (370, 470).

12. Seringue à vis (100) selon l'une quelconque des revendications précédentes,
**caractérisée par** une pointe de piston (380) disposée dans le corps de seringue (300), comprenant une première section (381), dont le diamètre extérieur est adapté à un diamètre intérieur de la douille (310) et qui présente de préférence un renfoncement (382) pour la prise avec une extrémité (220) de la tige de piston (200), sachant que la pointe de piston (380) comprend de préférence en outre une section (383) de forme tronconique, qui présente, au niveau de son extrémité d'alimentation (384), un diamètre extérieur qui est inférieur à un diamètre intérieur d'une ouverture de sortie (351) au niveau de l'extrémité de sortie (350).

13. Corps de seringue (300) pour une seringue à vis (100) servant à distribuer une matière selon l'une quelconque des revendications précédentes, comprenant
une douille (310) servant à recevoir une matière, sachant que la douille présente une extrémité de sortie (350) pour la sortie d'une matière se trouvant dans la douille (310) dans une direction de sortie (302) axiale et une extrémité de piston (360) servant à recevoir une tige de piston (200), et sachant que l'extrémité de piston (360) est réalisée de telle manière qu'une partie écrou (400) pour une seringue à vis (100) selon l'une quelconque des revendications précédentes peut être enfilée dans une direction d'enfilement (303) radiale sur l'extrémité de piston (360),
sachant que
- l'extrémité de piston (360) présente une face complémentaire (340), qui peut être amenée dans une complémentarité de forme avec une bride (440) réalisée au niveau de la partie écrou (400) de telle manière qu'un déplacement par coulissement axial relatif entre la partie écrou (400) et le corps de seringue (300) dans la direction de sortie (302) est empêché,
- l'extrémité de piston (360) présente des faces de maintien complémentaires (320a, b), qui peuvent être amenées dans une complémentarité de forme avec des faces de maintien (420a, b) réalisées au niveau de la partie écrou (400) de telle manière qu'un déplacement par coulissement axial relatif entre la partie écrou (400) et le corps de seringue (300) dans le sens inverse à la direction de sortie (302) est empêché, et
- les faces de maintien complémentaires (320a, b) peuvent être amenées dans une complémentarité de forme avec les faces de maintien (420a, b) de telle manière et sont inclinées par rapport à l'axe longitudinal (301)
- et forment, avec l'axe longitudinal (301), un angle qui est supérieur à 0° et inférieur à 90°, de telle manière
- qu'un écartement de la partie écrou (400) ou de parties de cette dernière vers l'extérieur, lequel est provoqué par une force appliquée lors d'un actionnement de la tige de piston (200), est empêché, et
- qu'un déplacement relatif radial orienté de manière perpendiculaire par rapport à la direction d'enfilement entre les faces de maintien inclinées de manière correspondante et les faces de maintien complémentaires est empêché.

14. Partie écrou (400) pour une seringue à vis (100) servant à distribuer une matière selon l'une quelconque des revendications 1 à 12, comprenant
un filetage intérieur (410) pour une prise avec un filetage extérieur (210) d'une tige de piston (200), sachant que la partie écrou (400) est réalisée de telle manière qu'elle peut être enfilée dans une direction d'enfilement (303) radiale sur une extrémité de piston (360) d'un corps de seringue (300) pour une seringue à vis (100) selon l'une quelconque des revendications 1 à 12,
sachant que
- la partie écrou (400) présente une bride (440), qui peut être amenée dans une complémentarité de forme avec une face complémentaire (340) réalisée au niveau d'une extrémité de piston (360) d'un corps de seringue (300) de telle manière qu'un déplacement par coulissement axial relatif entre la partie écrou (400) et le corps de seringue (300) en direction de sortie (302) est empêché,
- la partie écrou (400) présente des faces de maintien (420a, b), qui peuvent être amenées dans une complémentarité de forme avec des faces de maintien complémentaires (320a, b) réalisées au niveau d'une extrémité de piston (360) d'un corps de seringue (300) de telle manière qu'un déplacement par coulissement axial relatif entre la partie écrou (400) et le corps de seringue (300) dans le sens inverse de la direction de sortie (303) est empêché, et
- les faces de maintien (420a, b) peuvent être amenées dans une complémentarité de forme avec les faces de maintien complémentaires (320a, b) de telle manière et sont inclinées par rapport à l'axe longitudinal (301) de telle manière,
- et forment avec l'axe longitudinal (301) un angle qui est supérieur à 0° et inférieur à 90°, de telle manière
- qu'un écartement de la partie écrou (400) ou de parties de cette dernière vers l'extérieur, lequel est provoqué par une force appliquée lors d'un actionnement de la tige de piston (200), est empêché, et
- qu'un déplacement relatif radial orienté de manière perpendiculaire par rapport à la direction d'enfilement entre les faces de maintien et les faces de maintien complémentaires inclinées de manière correspondante est empêché.
